# EUROPEAN PATENT APPLICATION

(11) **EP 4 696 694 A1**
(43) Date of publication of application: **18.02.2026**
(21) Application number: 24194635.9
(22) Date of filing: 14.08.2024
(51) Int. Cl.: C07D 498/22, A61K 31/438, A61P 31/04

(54) **ANTIBIOTIC RIFABUTIN ANALOGS**

(71) Applicant: BioVersys AG, 4057 Basel (CH)
(72) Inventor: BANDERA, Monica, 74350 Villy-le-Bouveret (FR); DALE, Glenn E., 4053 Basel (CH); DRENEAU, Aurore, 59800 Lille (FR); GITZINGER, Marc, 4057 Basel (CH); HOFMANN, Line, 59000 Lille (FR); KHAN, Nawaz, 4057 Basel (CH); LOCIURO, Sergio, 4058 Basel (CH); Michelotti, Alessia, 59110 La Madeleine (FR); MILJKOVIC, Marija, 4057 Basel (CH); TREBOSC, Vincent, 68680 Kembs (FR); WILLAND, Nicolas, 59800 Lille (FR)
(74) Representative: Vossius & Partner Patentanwälte Rechtsanwälte mbB

(57) **Abstract**

The present invention relates to antibiotic rifabutin analogs of formula I 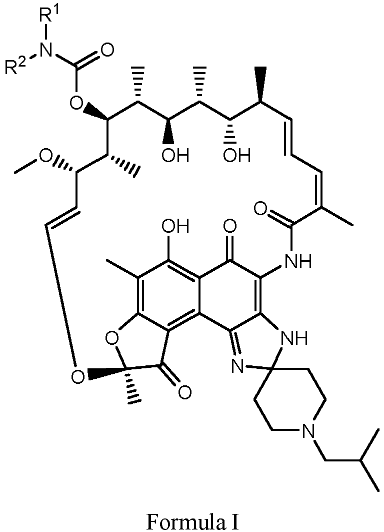 wherein:
R¹ is -H or -C₁-C₆ alkyl;
R² is a group of Formula II: 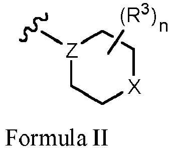 In some embodiments, the bacterial infection is caused by one or more bacterium belonging to a genus of non-tuberculous *Mycobacteria,* preferably *M. abscessus.*

## Description

The present invention relates to compounds and pharmaceutical compositions comprising the same for the treatment, amelioration and/or prevention of disease. In some embodiments, the disease is an infection, preferably a bacterial infection. In some embodiments, the bacterium belongs to a genus of non-tuberculous *Mycobacteria* (NTM), preferably *M. abscessus.*

### RELATED ART

Rifamycins such as rifabutin are known antibiotics with activity against a broad spectrum of pathogens such as *Clostridium spp., Enterococcus spp., Hemophilus spp.*, *Legionella spp., Mycobacterium spp.* (tuberculous and non-tuberculous Mycobacteria), *Neisseria spp., Staphylococcus spp., Streptococcus spp., Listeria monocytogenes, Moraxella catarrhalis, Bacillus spp., Bacteroides spp., Gardnerella vaginalis, Lactobacillus spp., Mobiluncus spp.*, *Helicobacter pylori, Campylobacter jejuni, Chlamydia trachomatis* and *Toxoplasma gondii* (Kunin, Clin. Infect. Dis., 1996; Farr and Mandell, Med. Clin. North. Am., 1982; Thomsberry et al., Rev. Infect. Dis., 1983; Hoover et al., Diagn. Microbiol. Infect. Dis., 1993; Kerry et al., J. Antimicrob. Chemother., 1975).

Rifabutin has been recently shown to have potent *in vitro* and *in vivo* activity against *Mycobacterium abscessus* (Aziz et al., Antimicrob. Agents Chemother., 2017; Dick et al., Antimicrob. Agents Chemother., 2020) and *Acinetobacter baumannii* (Luna et al., Nat. Microbiol., 2020; Trebosc et al., Drug Discov. Today, 2021; Trebosc et al., J. Antimicrob. Chemother., 2020). However, there remains a need for more effective rifamycins for the treatment of bacterial infections such as *M. abscessus* and *A. baumannii* infections.

### SUMMARY OF THE INVENTION

In one aspect, the present disclosure provides a compound of Formula I or a pharmaceutically acceptable salt, tautomer, solvate, hydrate or enantiomer thereof: 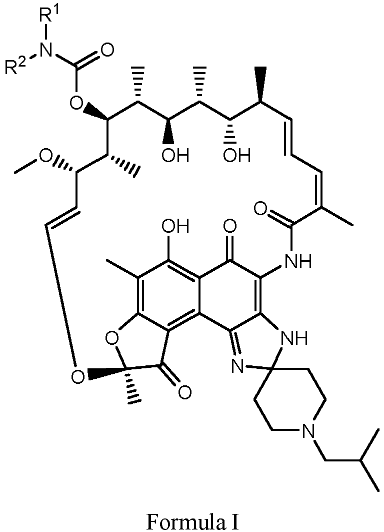 wherein:
R¹ is -H or -C₁-C₆ alkyl;
R² is a group of Formula II: 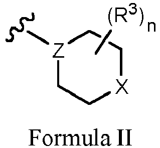 wherein
   n is an integer from 0 to 8;
   R³ is independently, at each occurrence, C₁-C₆ alkyl; or two R³, together with the carbon atoms to which they are attached, optionally combine to form a bridging alkylene, preferably a bridging C₁-C₂ alkylene;
   X is O or NR⁴;
   Z is CH or N;
   R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

In one aspect, the present invention provides a pharmaceutical composition comprising a compound as described herein, or a pharmaceutically acceptable salt thereof, and a pharmaceutically acceptable carrier. In some preferred embodiments, the pharmaceutical composition is effective for treating a disease, preferably an infection, more preferably a bacterial infection. In some preferred embodiments, the bacterial infection is caused by one or more bacterium belonging a genus of non-tuberculous *Mycobacteria,* preferably *M. abscessus.*

In one aspect, the present invention provides a compound of Formula I or a pharmaceutically acceptable salt thereof as described herein for use as a medicament.

In one aspect, the present invention provides a compound of Formula I or a pharmaceutically acceptable salt thereof as described herein for use in a method for treating a disease, preferably an infection, more preferably a bacterial infection in a subject. In some preferred embodiments, the bacterial infection is caused by one or more bacterium belonging a genus of non-tuberculous *Mycobacteria,* preferably *M. abscessus.*

In one aspect, the present invention provides a use of a compound or pharmaceutical composition comprising a compound as described herein in the manufacture of a medicament for treating a disease, preferably an infection, more preferably a bacterial infection. In some preferred embodiments, the bacterial infection is caused by one or more bacterium belonging a genus of non-tuberculous *Mycobacteria,* preferably *M. abscessus.*

In one aspect, the present invention provides a method of treating a disease, preferably an infection, more preferably a bacterial infection in a subject in need thereof, comprising administering to the subject a therapeutically effective amount of a compound as described herein or a pharmaceutically acceptable salt thereof. In some preferred embodiments, the bacterial infection is caused by one or more bacterium belonging a genus of non-tuberculous *Mycobacteria,* preferably *M. abscessus.*

The present invention provides rifabutin analogs that are modified at the C25 position and pharmaceutical compositions thereof. The inventive compounds exhibit broad antibacterial activity against both smooth and rough variants of *M. abscessus.* Additional features and advantages of the present technology will be apparent to one of skill in the art upon reading the Detailed Description, below.

### DETAILED DESCRIPTION OF THE INVENTION

The present invention provides analogs of rifabutin that are effective in treating bacterial infections, preferably bacterial infections caused by one or more bacterium belonging to a genus of non-tuberculous *Mycobacteria,* preferably *M. abscessus.*

The details of the technology are set forth in the accompanying description below. Although methods and materials similar or equivalent to those described herein can be used in the practice or testing of the present technology, illustrative methods and materials are now described. Other features, objects, and advantages of the invention will be apparent from the description and from the claims.

### Definitions

Unless defined otherwise, all technical and scientific terms used herein have the same meanings as commonly understood by one of ordinary skill in the art to which this invention belongs.

The articles "a" and "an" are used in this disclosure to refer to one or more than one (i.e., to at least one) of the grammatical object of the article unless the context clearly dictates otherwise. By way of example, "an element" means one element or more than one element.

The term "and/or" is used in this disclosure to mean either "and" or "or" unless indicated otherwise.

The term "optionally substituted" is understood to mean that a given chemical moiety (e.g. an alkyl group) can (but is not required to) be bonded other substituents (e.g. heteroatoms). For instance, an alkyl group that is optionally substituted can be a fully saturated alkyl chain (i.e. a pure hydrocarbon). Alternatively, the same optionally substituted alkyl group can have substituents different from hydrogen. For instance, it can, at any point along the chain be bonded to a halogen atom, a hydroxyl group, or any other substituent described herein. Thus, the term "optionally substituted" means that a given chemical moiety has the potential to contain other functional groups, but does not necessarily have any further functional groups.

The term "alkyl" refers to a straight or branched chain saturated hydrocarbon. C₁-C₆ alkyl groups contain 1 to 6 carbon atoms. Examples of a-C₁-C₆ alkyl group include, but are not limited to, methyl, ethyl, propyl, butyl, pentyl, isopropyl, isobutyl, sec-butyl and tert-butyl, isopentyl and neopentyl.

The terms "alkylene" or "alkylenyl," as used herein, refer to a straight or branched hydrocarbon chain bi-radical derived from alkyl, as defined herein, wherein one hydrogen of said alkyl is cleaved off generating the second radical of said alkylene. Examples of alkylene are, by way of illustration, -CH₂-, -CH₂-CH₂-, -CH(CH₃)-, -CH₂-CH₂-CH₂-, -CH(CH₃)-CH₂-, or -CH(CH₂CH₃)-.

"C₁-C₆ alkoxy", as used herein, refers to straight chain or branched saturated -O-C₁-C₆ hydrocarbon which may be, for example, methoxy, ethoxy, *n*-propoxy, isopropoxy, *n*-butoxy, isobutoxy, *sec*-butoxy, *tert*-butoxy, straight or branched pentoxy, straight or branched hexyloxy, straight or branched heptyloxy, or straight or branched octyloxy. Preferably, C₁-C₆ alkoxy is C₁-C₄ alkoxy. A "C₁-C₆ alkoxy" group can also be represented as "-O-C₁-C₆ alkyl."

The term "cycloalkyl" means monocyclic or bicyclic saturated carbon rings containing 3-10 carbon atoms. Examples of cycloalkyl groups include, without limitations, cyclopropyl, cyclobutyl, cyclopentyl, cyclohexyl, cycloheptyl, cyclooctyl, cyclononyl, cyclodecyl, octahydro-1H-indene, and decalin.

The terms "heterocyclyl" or "heterocycloalkyl" or "heterocycle" refer to monocyclic or polycyclic saturated or partially saturated 3 to 15-membered rings containing carbon and heteroatoms taken from O, N, and S (preferably O and N) and wherein at least one ring does not comprise delocalized π electrons (aromaticity) shared among the ring carbon or heteroatoms. A 3-10 membered heterocycloalkyl group contains between 3 and 10 atoms.

Heterocyclyl rings include, but are not limited to, oxetanyl, azetadinyl, tetrahydrofuranyl, pyrrolidinyl, oxazolinyl, oxazolidinyl, thiazolinyl, thiazolidinyl, pyranyl, thiopyranyl, tetrahydropyranyl, piperidinyl, morpholinyl, thiomorpholinyl, thiomorpholinyl S-oxide, thiomorpholinyl S-dioxide, piperazinyl, azepinyl, oxepinyl, [1,4]diazepane, [1,2]diazepane, decahydro-[1,6]naphthyridine diazepinyl, octahydro-pyrrolo[1,2-a]pyrazinyl, octahydro-pyrrolo[3,2-c]pyridinyl, and 2,5-diaza-bicyclo[2.2.1]heptanyl.

A heterocyclyl or heterocycloalkyl ring can also be fused or bridged, e.g., can be a bicyclic or tricyclic ring.

As used herein, the term "tetrahydropyran" is understood to be a six-membered heterocycloalkyl of the formula: 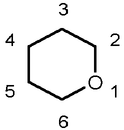 wherein the atom numbering is shown in the formula above. When R² is tetrahydropyran, the expression "bonded at a 4-carbon of said tetrahydropyran" is understood to mean that the R² tetrahydropyran is bonded to the amide nitrogen in Formula I via the 4-carbon as shown below:

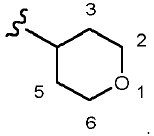

As used herein, the term "morpholine" is understood to be a six-membered heterocycloalkyl of the formula: 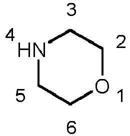 wherein the atom numbering is shown in the formula above. When R² is morpholine, the expression "bonded at a 4-nitrogen of said morpholine" is understood to mean that the R² morpholine is bonded to the amide nitrogen in Formula I via the 4-nitrogen as shown below:

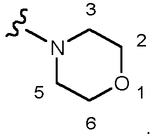

As used herein, the term "8-oxabicyclo[3.2.1]octane" is understood to be an 8-membered bicyclic heterocycle of the formula: 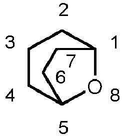 wherein the atom numbering is shown in the formula above. When R² is 8-oxabicyclo[3.2.1]octane, the expression "bonded at a 3-carbon of said 8-oxabicyclo[3.2.1]octane" is understood to mean that the R² 8-oxabicyclo[3.2.1]octane is bonded to the amide nitrogen in Formula I via the 3-carbon as shown below:

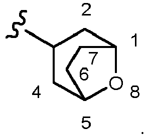

As used herein, the term "piperidine" is understood to be a 6-membered heterocyclic ring of the formula: 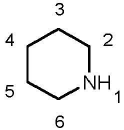 wherein the atom numbering is shown in the formula above. When R² is piperidine, the expressions "said piperidine is bonded at a 4-carbon of said piperidine" and "[said] piperidine is independently substituted at the 1-nitrogen with R⁴" are understood to mean that the R² piperidine is bonded to the amide nitrogen in Formula I via the 4-carbon and substituted with R⁴ at the 1-nitrogen as shown below:

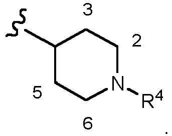

As used herein, the term "2-azabicyclo[2.2.1]heptane" is understood to be a 7-membered bicyclic heterocycle of the formula: 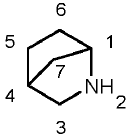 wherein the atom numbering is shown in the formula above. When R² is 2-azabicyclo[2.2.1]heptane, the expressions "said 2-azabicyclo[2.2.1]heptane is bonded at a 5-carbon of said 2-azabicyclo[2.2.1]heptane" and "[said] 2-azabicyclo[2.2.1]heptane is independently substituted at the 2-nitrogen with R⁴" are understood to mean that the R² 2-azabicyclo[2.2.1]heptane is bonded to the amide nitrogen in Formula I via the 5-carbon and substituted with R⁴ at the 2-nitrogen as shown below:

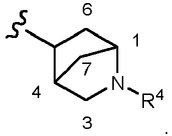

The invention also includes pharmaceutical compositions comprising an effective amount of a disclosed compound and a pharmaceutically acceptable carrier. Representative "pharmaceutically acceptable salts" include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, hydroiodide, sethionate, lactate, lactobionate, laurate, magnesium, malate, maleate, mandelate, mesylate, methylbromide, methylnitrate, methyl sulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "stereoisomers" refers to the set of compounds which have the same number and type of atoms and share the same bond connectivity between those atoms, but differ in three-dimensional structure. The term "stereoisomer" refers to any member of this set of compounds.

The term "diastereomers" refers to the set of stereoisomers which cannot be made superimposable by rotation around single bonds. For example, cis- and trans-double bonds, *endo-* and *exo*-substitution on bicyclic ring systems, and compounds containing multiple stereogenic centers with different relative configurations are considered to be diastereomers. The term "diastereomer" refers to any member of this set of compounds. In some examples presented, the synthetic route may produce a single diastereomer or a mixture of diastereomers. In some cases these diastereomers were separated and in other cases a wavy bond is used to indicate the structural element where configuration is variable.

The term "enantiomers" refers to a pair of stereoisomers which are non-superimposable mirror images of one another. The term "enantiomer" refers to a single member of this pair of stereoisomers. The term "racemic" refers to a 1:1 mixture of a pair of enantiomers.

The term "tautomers" refers to a set of compounds that have the same number and type of atoms, but differ in bond connectivity and are in equilibrium with one another. A "tautomer" is a single member of this set of compounds. Typically a single tautomer is drawn but it is understood that this single structure is meant to represent all possible tautomers that might exist. Examples include enol-ketone tautomerism. When a ketone is drawn it is understood that both the enol and ketone forms are part of the present disclosure.

The term "solvate" refers to a complex of variable stoichiometry formed by a solute and solvent. Such solvents for the purpose of the invention may not interfere with the biological activity of the solute. Examples of suitable solvents include, but are not limited to, water, MeOH, EtOH, and AcOH. Solvates wherein water is the solvent molecule are typically referred to as "hydrates." Hydrates include compositions containing stoichiometric amounts of water, as well as compositions containing variable amounts of water.

An "effective amount" when used in connection with a compound is an amount effective for treating or preventing a disease in a subject as described herein.

The term "carrier", as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a disclosed compound or pharmaceutically acceptable salt of the disclosed compound or a composition to a subject, or administering a prodrug derivative or analog of the compound or pharmaceutically acceptable salt of the compound or composition to the subject, which can form an equivalent amount of active compound within the subject's body.

A "patient" or "subject" is a mammal, e.g., a human, mouse, rat, guinea pig, dog, cat, horse, cow, pig, or non-human primate, such as a monkey, chimpanzee, baboon or rhesus.

### Compounds of the Invention

In one aspect, the present disclosure provides a compound of Formula I or a pharmaceutically acceptable salt, tautomer, solvate, hydrate or enantiomer thereof: 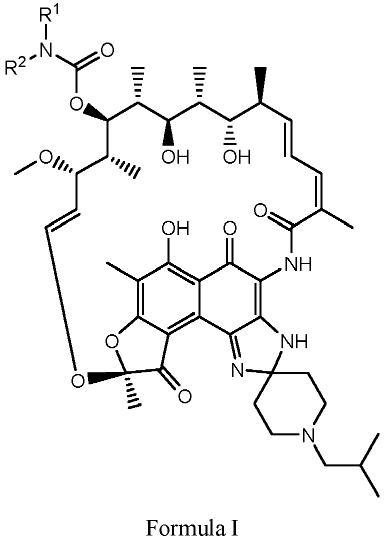 wherein:
R¹ is -H or -C₁-C₆ alkyl;
R² is a group of Formula II: 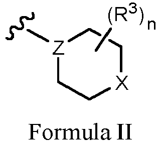 wherein
   n is an integer from 0 to 8;
   R³ is independently, at each occurrence, C₁-C₆ alkyl; or two R³, together with the carbon atoms to which they are attached, optionally combine to form a bridging alkylene, preferably a bridging C₁-C₂ alkylene;
   X is O or NR⁴;
   Z is CH or N;
   R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

In one aspect, the present disclosure provides a compound of Formula I or a pharmaceutically acceptable salt, tautomer, solvate, hydrate or enantiomer thereof: 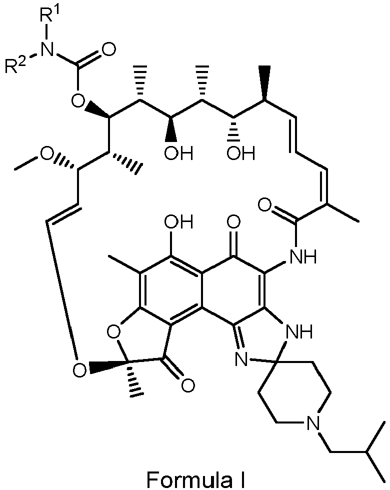 wherein:
R¹ is -H or -C₁-C₆ alkyl;
R² is selected from the group consisting of tetrahydropyran, morpholine, 8-oxabicyclo[3.2.1]octane, piperidine, and 2-azabicyclo[2.2.1]heptane;
wherein said tetrahydropyran is bonded at a 4-carbon of said tetrahydropyran; said morpholine is bonded at a 4-nitrogen of said morpholine; said 8-oxabicyclo[3.2.1]octane is bonded at a 3-carbon of said 8-oxabicyclo[3.2.1]octane; said piperidine is bonded at a 4-carbon of said piperidine; and said 2-azabicyclo[2.2.1]heptane is bonded at a 5-carbon of said 2-azabicyclo [2.2.1]heptane;
wherein each tetrahydropyran, morpholine, and 8-oxabicyclo[3.2.1]octane is independently optionally substituted with one or more R³;
wherein each piperidine and 2-azabicyclo[2.2.1]heptane is independently optionally substituted with one or more R³, wherein each piperidine is independently substituted at the 1-nitrogen with R⁴, and wherein each 2-azabicyclo[2.2.1]heptane is independently substituted at the 2-nitrogen with R⁴;
R³ is independently, at each occurrence, C₁-C₆ alkyl;
R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

In some embodiments, R¹ is hydrogen. In some embodiments, R¹ is H or C₁-C₄ alkyl. In some embodiments, R¹ is H or C₁-C₂ alkyl. In some embodiments, R¹ is H or methyl. In some embodiments, R¹ is C₁-C₄ alkyl. In some embodiments, R¹ is C₁-C₂ alkyl. In some embodiments, R¹ is methyl.

In some embodiments, n is an integer from 0 to 4, preferably from 0 to 2. In some embodiments, n is an integer from 0 to 6. In some embodiments, n is an integer from 0 to 4. In some embodiments, n is an integer from 0 to 2. In some embodiments, n is an integer from 0 to 1. In some embodiments, n is 0. In some embodiments, n is 1. In some embodiments, n is 2. In some embodiments, n is 3. In some embodiments, n is 4. In some embodiments, n is 5. In some embodiments, n is 6. In some embodiments, n is 7. In some embodiments, n is 8.

In some embodiments, said R³ is independently, at each occurrence, C₁-C₄ alkyl. In some embodiments, R³ is independently, at each occurrence, C₁-C₂ alkyl. In some embodiments, R³ is independently, at each occurrence, methyl. In some embodiments, R³ is independently, at each occurrence, C₁-C₂ alkyl, and n is an integer from 0 to 2. In some embodiments, R³ is independently, at each occurrence, methyl, and n is an integer from 0 to 2.

In some embodiments, Z is CH. In some embodiments, Z is N.

In some embodiments, X is O.

In some embodiments, X is O and Z is CH.

In some embodiments, X is O and Z is N.

In some embodiments, X is O;
n is an integer from 0 to 4; and
R³ is independently, at each occurrence, C₁-C₄ alkyl; or two R³, together with the carbon atoms to which they are attached, optionally combine to form a bridging alkylene, preferably a C₁-C₂ bridging alkylene.

In some embodiments, X is O;
n is an integer from 0 to 2;
R³ is independently, at each occurrence, C₁-C₂ alkyl; or two R³, together with the carbon atoms to which they are attached, optionally combine to form a bridging alkylene, preferably a C₁-C₂ bridging alkylene.

In some embodiments, X is O;
n is an integer from 0 to 4;
R¹ is independently, at each occurrence, H or C₁-C₄ alkyl; and
R³ is independently, at each occurrence, C₁-C₄ alkyl; or two R³, together with the carbon atoms to which they are attached, optionally combine to form a C₁-C₂ bridging alkylene.

In some embodiments, X is O;
n is an integer from 0 to 2;
R¹ is independently, at each occurrence, H or C₁-C₂ alkyl; and
R³ is independently, at each occurrence, C₁-C₂ alkyl.

In some embodiments, X is O;
n is an integer from 0 to 2;
R¹ is independently, at each occurrence, H or C₁-C₂ alkyl; and
R³ is independently, at each occurrence, C₁-C₂ alkyl; or two R³, together with the carbon atoms to which they are attached, optionally combine to form a C₁-C₂ bridging alkylene.

In some embodiments, X is NR⁴.

In some embodiments, X is NR⁴ and Z is CH.

In some embodiments, X is NR⁴ and Z is N.

In some embodiments, X is NR⁴;
n is an integer from 0 to 4;
R³ is independently, at each occurrence, C₁-C₄ alkyl; or two R³, together with the carbon atoms to which they are attached, optionally combine to form a C₁-C₂ bridging alkylene; and
R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

In some embodiments, X is NR⁴;
n is 0 or 2;
when n is 2, both R³, together with the carbon atoms to which they are attached, combine to form a C₁-C₂ bridging alkylene; and
R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

In some embodiments, X is NR⁴;
n is 0 or 2;
when n is 2, both R³, together with the carbon atoms to which they are attached, combine to form a C₁-C₂ bridging alkylene; and
R⁴ is independently, at each occurrence, selected from 3- to 5-membered heterocycloalkyl and -C(O)C₁-C₆ alkyl, wherein said C₁-C₆ alkyl is optionally substituted with one or more C₁-C₆ alkoxy.

In some embodiments, R² is selected from the group consisting of tetrahydropyran, morpholine, 8-oxabicyclo[3.2.1]octane, piperidine, and 2-azabicyclo[2.2.1]heptane;
wherein said tetrahydropyran is bonded at a 4-carbon of said tetrahydropyran; said morpholine is bonded at a 4-nitrogen of said morpholine; said 8-oxabicyclo[3.2.1]octane is bonded at a 3-carbon of said 8-oxabicyclo[3.2. 1]octane; said piperidine is bonded at a 4-carbon of said piperidine; and said 2-azabicyclo[2.2.1]heptane is bonded at a 5-carbon of said 2-azabicyclo [2.2.1]heptane;
wherein each tetrahydropyran, morpholine, 8-oxabicyclo[3.2.1]octane, piperidine, and 2-azabicyclo[2.2.1]heptane is independently optionally substituted with one or more R³;
wherein each piperidine is independently substituted at the 1-nitrogen with R⁴, and wherein each 2-azabicyclo[2.2. 1]heptane is independently substituted at the 2-nitrogen with R⁴;
R³ is independently, at each occurrence, C₁-C₆ alkyl;
R⁴ is independently, at each occurrence, selected from 3- to 5-membered heterocycloalkyl, -C(O)C₁-C₆ alkyl, wherein said C₁-C₆ alkyl is optionally substituted with one or more C₁-C₆ alkoxy, and -C₂-C₆ alkyl, wherein said -C₂-C₆ alkyl is substituted with one or more C₁-C₆ alkoxy.

In some embodiments, R² is selected from the group consisting of tetrahydropyran, morpholine, 8-oxabicyclo[3.2.1]octane, piperidine, and 2-azabicyclo[2.2.1]heptane;
wherein said tetrahydropyran is bonded at a 4-carbon of said tetrahydropyran; said morpholine is bonded at a 4-nitrogen of said morpholine; said 8-oxabicyclo[3.2.1]octane is bonded at a 3-carbon of said 8-oxabicyclo[3.2. 1]octane; said piperidine is bonded at a 4-carbon of said piperidine; and said 2-azabicyclo[2.2.1]heptane is bonded at a 5-carbon of said 2-azabicyclo [2.2.1]heptane;
wherein each tetrahydropyran, morpholine, 8-oxabicyclo[3.2.1]octane, piperidine, and 2-azabicyclo[2.2.1]heptane is independently optionally substituted with one or more R³;
wherein each piperidine is independently substituted at the 1-nitrogen with R⁴, and wherein each 2-azabicyclo[2.2. 1]heptane is independently substituted at the 2-nitrogen with R⁴;
R³ is independently, at each occurrence, C₁-C₆ alkyl;
R⁴ is independently, at each occurrence, selected from 3- to 5-membered heterocycloalkyl and -C(O)C₁-C₆ alkyl, wherein said C₁-C₆ alkyl is optionally substituted with one or more C₁-C₆ alkoxy.

In some embodiments, R² is selected from the group consisting of tetrahydropyran, morpholine, 8-oxabicyclo[3.2.1]octane, piperidine, and 2-azabicyclo[2.2.1]heptane;
wherein said tetrahydropyran is bonded at a 4-carbon of said tetrahydropyran; said morpholine is bonded at a 4-nitrogen of said morpholine; said 8-oxabicyclo[3.2.1]octane is bonded at a 3-carbon of said 8-oxabicyclo[3.2.1]octane; said piperidine is bonded at a 4-carbon of said piperidine; and said 2-azabicyclo[2.2.1]heptane is bonded at a 5-carbon of said 2-azabicyclo[2.2.1]heptane;
wherein each tetrahydropyran, morpholine, and 8-oxabicyclo[3.2.1]octane is independently optionally substituted with one or more R³;
wherein each piperidine is independently substituted at the 1-nitrogen with R⁴, and wherein each 2-azabicyclo[2.2.1]heptane is independently substituted at the 2-nitrogen with R⁴;
R³ is independently, at each occurrence, C₁-C₆ alkyl;
R⁴ is independently, at each occurrence, selected from 3- to 5-membered heterocycloalkyl and -C(O)C₁-C₆ alkyl, wherein said C₁-C₆ alkyl is optionally substituted with one or more C₁-C₆ alkoxy.

In some embodiments, R² is selected from the group consisting of tetrahydropyran, morpholine, 8-oxabicyclo[3.2.1]octane, piperidine, and 2-azabicyclo[2.2.1]heptane;
wherein said tetrahydropyran is bonded at a 4-carbon of said tetrahydropyran; said morpholine is bonded at a 4-nitrogen of said morpholine; said 8-oxabicyclo[3.2.1]octane is bonded at a 3-carbon of said 8-oxabicyclo[3.2.1]octane; said piperidine is bonded at a 4-carbon of said piperidine; and said 2-azabicyclo[2.2.1]heptane is bonded at a 5-carbon of said 2-azabicyclo [2.2.1]heptane;
wherein each tetrahydropyran, morpholine, and 8-oxabicyclo[3.2.1]octane is independently optionally substituted with one or more R³;
wherein each piperidine is independently substituted at the 1-nitrogen with R⁴, and wherein each 2-azabicyclo[2.2.1]heptane is independently substituted at the 2-nitrogen with R⁴;
R³ is independently, at each occurrence, C₁-C₄ alkyl, preferably C₁-C₂ alkyl;
R⁴ is independently, at each occurrence, selected from 3- to 5-membered heterocycloalkyl and -C(O)C₁-C₄ alkyl, wherein said C₁-C₄ alkyl is optionally substituted with one or more C₁-C₄ alkoxy, preferably C₁-C₂ alkoxy.

In some embodiments, R² is selected from the group consisting of tetrahydropyran, morpholine, and 8-oxabicyclo[3.2.1]octane;
wherein said tetrahydropyran is bonded at a 4-carbon of said tetrahydropyran; said morpholine is bonded at a 4-nitrogen of said morpholine; and said 8-oxabicyclo[3.2.1]octane is bonded at a 3-carbon of said 8-oxabicyclo[3.2.1]octane;
wherein each tetrahydropyran, morpholine, and 8-oxabicyclo[3.2.1]octane is independently optionally substituted with one or more R³; and
R³ is independently, at each occurrence, C₁-C₆ alkyl.

In some embodiments, R² is piperidine or 2-azabicyclo[2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2.1]heptane is independently substituted at the 2-nitrogen with R⁴;
R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

In preferred embodiments, R² is piperidine, wherein said piperidine is bonded at the 4-carbon. In preferred embodiments, R² is azabicyclo[2.2.1]heptane, wherein said azabicyclo[2.2.1]heptane is bonded at the 5-carbon.

In preferred embodiments, R² is piperidine, wherein said piperidine is bonded at the 4-carbon and wherein said piperidine is substituted with R⁴ at the 1-nitrogen. In preferred embodiments, R² is azabicyclo[2.2.1]heptane, wherein said azabicyclo[2.2.1]heptane is bonded at the 5-carbon and wherein said azabicyclo[2.2.1]heptane is substituted with R⁴ at the 2-nitrogen.

In some embodiments, R² is piperidine or 2-azabicyclo[2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2.1]heptane is independently substituted at the 2-nitrogen with R⁴;
R⁴ is independently, at each occurrence, selected from 3- to 5-membered heterocycloalkyl; and -C(O)C₁-C₆ alkyl, wherein said C₁-C₆ alkyl is optionally substituted with one or more C₁-C₆ alkoxy.

In some embodiments, R² is piperidine or 2-azabicyclo[2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2.1]heptane is independently substituted at the 2-nitrogen with R⁴;
R⁴ is independently, at each occurrence, selected from -C₂-C₆ alkyl, wherein said C₂-C₆ alkyl is substituted with one or more C₁-C₆ alkoxy; -CH₂-tetrahydropyran; and cyclopropyl.

In some embodiments, R² is piperidine, wherein said piperidine is bonded at a 4-carbon of said piperidine, wherein said piperidine is independently substituted at the 1-nitrogen with R⁴,
R⁴ is independently, at each occurrence, selected from 3- to 5-membered heterocycloalkyl; and -C(O)C₁-C₄ alkyl, wherein said C₁-C₄ alkyl is optionally substituted with one or more C₁-C₄ alkoxy.

In some embodiments, R² is piperidine, wherein said piperidine is bonded at a 4-carbon of said piperidine, wherein said piperidine is independently substituted at the 1-nitrogen with R⁴,
R⁴ is independently, at each occurrence, selected from -C₄ heterocycloalkyl; and - C(O)C₁-C₄ alkyl, wherein said C₁-C₄ alkyl is optionally substituted with one or more C₁-C₂ alkoxy.

In some embodiments, R² is piperidine, wherein said piperidine is bonded at a 4-carbon of said piperidine, wherein said piperidine is independently substituted at the 1-nitrogen with R⁴,
R⁴ is independently, at each occurrence, selected from oxetane, preferably 3-oxetane; and -C(O)C₁-C₄ alkyl, wherein said C₁-C₄ alkyl is optionally substituted with one or more C₁-C₄ alkoxy, preferably C₁-C₂ alkoxy.

In some embodiments, R² is piperidine or 2-azabicyclo[2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2. 1]heptane is independently substituted at the 2-nitrogen with R⁴;
wherein R⁴ is -C₂-C₆ alkyl, wherein said C₂-C₆ alkyl is substituted with one or more C₁-C₆ alkoxy.

In some embodiments, R² is piperidine,
wherein said piperidine is bonded at a 4-carbon of said piperidine, wherein said piperidine is independently substituted at the 1-nitrogen with R⁴;
wherein R⁴ is -C₂-C₄ alkyl, wherein said C₂-C₄ alkyl is substituted with one or more C₁-C₂ alkoxy.

In some embodiments, R² is piperidine,
wherein said piperidine is bonded at a 4-carbon of said piperidine, wherein said piperidine is independently substituted at the 1-nitrogen with R⁴;
wherein R⁴ is -CH₂-tetrahydropyran.

In some embodiments, R² is piperidine,
wherein said piperidine is bonded at a 4-carbon of said piperidine, wherein said piperidine is independently substituted at the 1-nitrogen with R⁴;
wherein R⁴ is cyclopropyl.

In one aspect, the present disclosure provides a pharmaceutical composition comprising at least one compound according to any of the preceding claims, or a pharmaceutically acceptable salt, tautomer, solvate or hydrate thereof, and a pharmaceutically acceptable excipient.

In one aspect, the present disclosure provides a compound as disclosed herein or a pharmaceutically acceptable salt, tautomer, solvate or hydrate thereof, for use as a medicament.

In one aspect, the present disclosure provides a compound as disclosed herein or a pharmaceutically acceptable salt, tautomer, solvate or hydrate thereof, for use in a method of preventing or treating a disease in a subject, preferably wherein said disease is an infection, further preferably a bacterial infection, yet further preferably a bacterial infection caused by one or more bacterium belonging to a genus of non-tuberculous *Mycobacteria,* preferably *M. abscessus.*

In one or more embodiments, R¹ and R² of a compound of the present invention can form one of the structures selected from Table 1, below, wherein the connectivity to the remainder of Formula I is shown below:

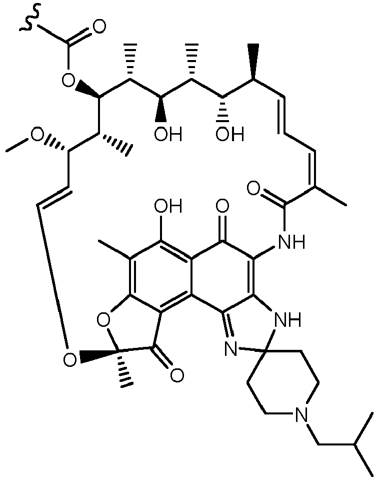

**Table 1. Embodiments of -NR¹R²**

| Compound | Structure | Compound | Structure |
|---|---|---|---|
| 1 | 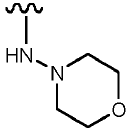 | 2 | 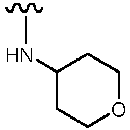 |
| 3 | 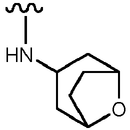 | 4 | 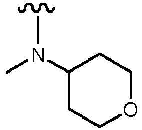 |
| 5 | 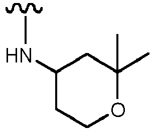 | 6 | 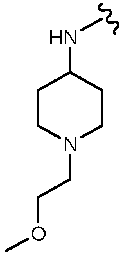 |
| 7 | 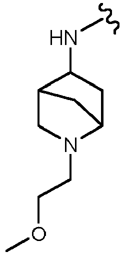 | 8 | 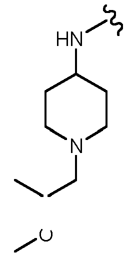 |
| 9 | 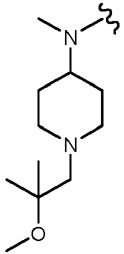 | 10 | 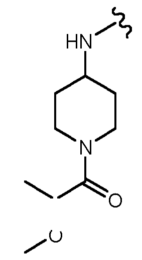 |
| 11 | 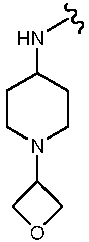 | 12 | 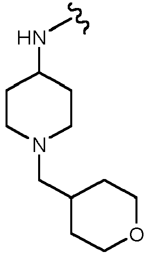 |
| 13 | 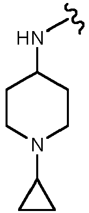 | | |

In some embodiments, the compound is a compound as shown below in Table 2, or a pharmaceutically acceptable salt thereof.

**Table 2. Embodied Compounds**

| Cmpd. | Structure | IUPAC Name |
|---|---|---|
| 1 | 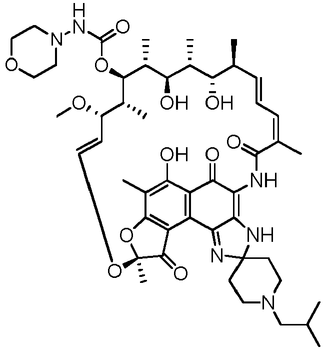 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-morpholinocarbamate |
| 2 | 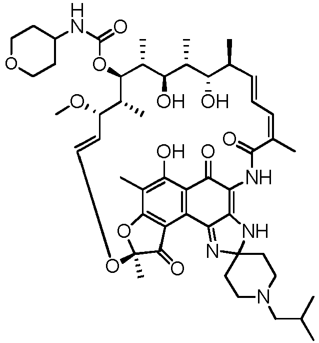 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-tetrahydropyran-4-ylcarbamate |
| 3 | 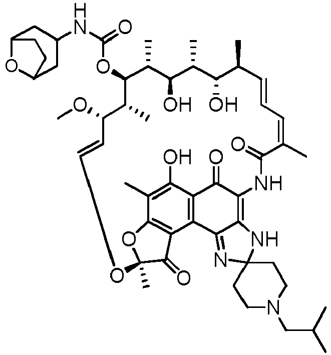 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1(31),2,4,9,19,21,25,29-octaene-28,4'-piperidine]-13-yl] N-(8-oxabicyclo[3.2.1]octan-3-yl)carbamate |
| 4 | 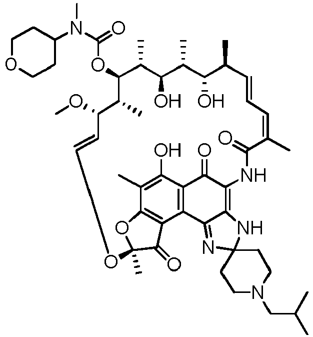 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-methyl-N-tetrahydropyran-4-yl-carbamate |
| 5 | 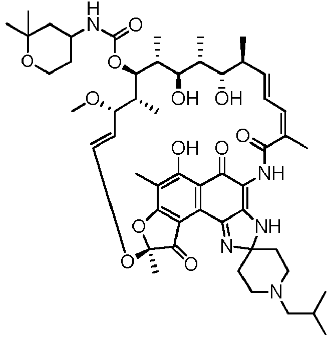 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tntriaconta-1(31),2,4,9,19,21,25,29-octaene-28,4'-piperidine]-13-yl] N-(2,2-dimethyltetrahydropyran-4-yl)carbamate |
| 6 | 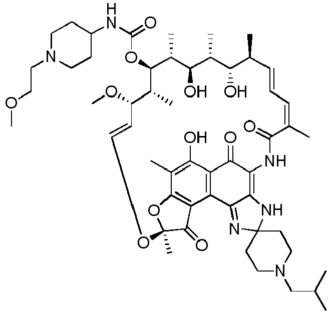 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-[1-(2-methoxyethyl)-4-piperidyl]carbamate |
| 7 | 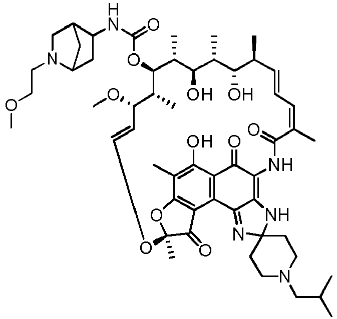 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-[2-(2-methoxyethyl)-2-azabicyclo[2.2.1]heptan-5-yl]carbamate |
| 8 | 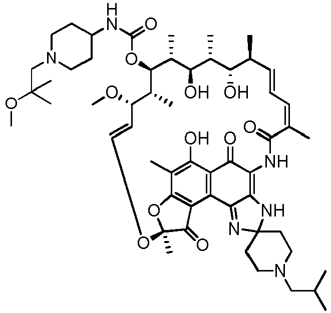 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-[1-(2-methoxy-2-methyl-propyl)-4-piperidyl] carbamate |
| 9 | 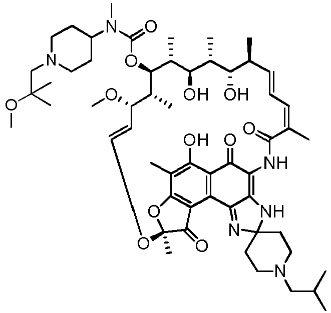 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-[1-(2-methoxy-2-methyl-propyl)-4-piperidyl]-N-methyl-carbamate |
| 10 | 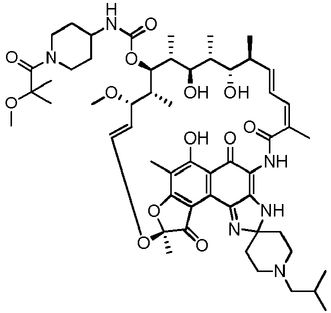 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-[1-(2-methoxy-2-methyl-propanoyl)-4-piperidyl] carbamate |
| 11 | 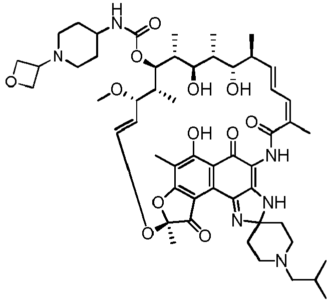 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6. 1. 14, 7.05,31.026,30] tritriaconta-1 (31),2,4,9,19,21,25,29-octaene-28,4'-piperidine]-13-yl] N-[1-(oxetan-3-yl)-4-piperidyl]carbamate |
| 12 | 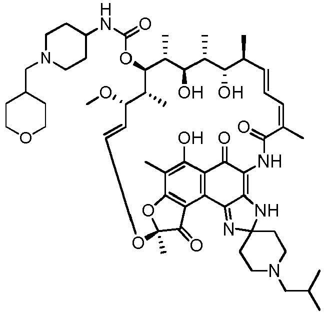 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-[1-(tetrahydropyran-4-ylmethyl)-4-piperidyl] carbamate |
| 13 | 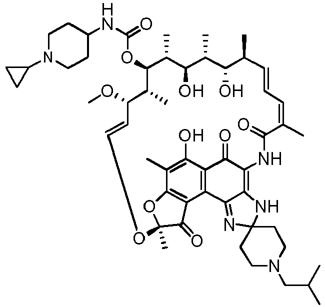 | [(7S,9E,11S,12R,13S,14R,15R,16R,17S,18 S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30] tritriaconta-1 (31),2,4,9,19,21,25,29-octaene-28,4'-piperidine]-13-yl] N-(1-cyclopropyl-4-piperidyl)carbamate |

In some embodiments, the compound is selected from the group consisting of Compounds 1, 2, 3, 4, 5, 6, 7, 8, 9, 10, 11, 12, and 13, or a pharmaceutically acceptable salt thereof.

In some embodiments, the compound is selected from the group consisting of Compounds 1, 2, 3, 4, 5, 10, and 11, or a pharmaceutically acceptable salt thereof.

In some embodiments, the compound is selected from the group consisting of Compounds 1, 2, 3, 4, and 5, or a pharmaceutically acceptable salt thereof.

In some embodiments, the compound is selected from the group consisting of Compounds 10 and 11, or a pharmaceutically acceptable salt thereof.

In some embodiments, the compound of Formula I is Compound 1, or a pharmaceutically acceptable salt thereof. In some embodiments, the compound of Formula I is Compound 2, or a pharmaceutically acceptable salt thereof. In some embodiments, the compound of Formula I is Compound 3, or a pharmaceutically acceptable salt thereof. In some embodiments, the compound of Formula I is Compound 4, or a pharmaceutically acceptable salt thereof. In some embodiments, the compound of Formula I is Compound 5, or a pharmaceutically acceptable salt thereof. In some embodiments, the compound of Formula I is Compound 6, or a pharmaceutically acceptable salt thereof. In some embodiments, the compound of Formula I is Compound 7, or a pharmaceutically acceptable salt thereof. In some embodiments, the compound of Formula I is Compound 8, or a pharmaceutically acceptable salt thereof. In some embodiments, the compound of Formula I is Compound 9, or a pharmaceutically acceptable salt thereof. In some embodiments, the compound of Formula I is Compound 10, or a pharmaceutically acceptable salt thereof. In some embodiments, the compound of Formula I is Compound 11, or a pharmaceutically acceptable salt thereof. In some embodiments, the compound of Formula I is Compound 12, or a pharmaceutically acceptable salt thereof. In some embodiments, the compound of Formula I is Compound 13, or a pharmaceutically acceptable salt thereof.

In one aspect, the present invention provides a pharmaceutical composition comprising at least one compound according to the present invention, or a pharmaceutically acceptable salt, tautomer, solvate or hydrate thereof, and a pharmaceutically acceptable excipient.

### Methods of Synthesizing the Disclosed Compounds

The compounds of the present invention may be made by a variety of methods, including standard chemistry. The methods include but are not limited to the methods described in the suitable synthetic routes depicted in the schemes given below.

The compounds of the present invention may be prepared by methods known in the art of organic synthesis as set forth in part by the following synthetic schemes and examples. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of compounds of the present invention.

Those skilled in the art will recognize if a stereocenter exists in the compounds of Formula I. Accordingly, the present invention includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley - lnterscience, 1994).

The compounds described herein may be made from commercially available starting materials or synthesized using known organic, inorganic, and/or enzymatic processes.

### Preparation of Compounds

Compounds of the present invention can be synthesized by following the steps outlined in General Schemes 1, 2 and 3. Starting materials are either commercially available or made by known procedures in the reported literature or as illustrated.

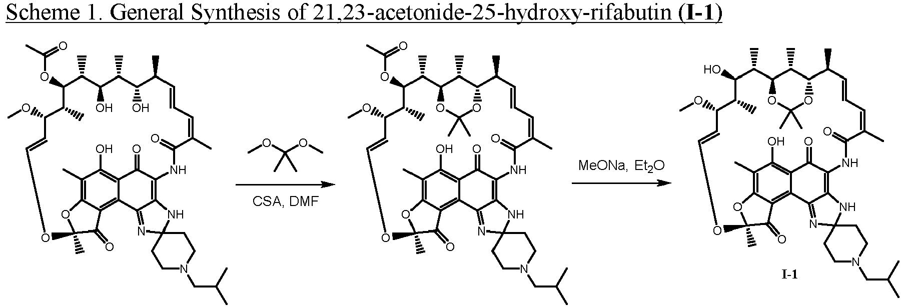

A general method for the preparation of 21,23-acetonide-25-hydroxy-rifabutin **I-1** is shown above in Scheme 1. Protection of the C21 and C23 hydroxy groups using a suitable protecting group such as dimethoxy propane and camphorsulphonic acid in a solvent, e.g., DMF afforded an acetal-protected rifabutin. De-acetylation of the protected rifabutin using a basic solution such as sodium methoxide in ether afforded the C25-hydroxy, C21-C23-protected rifabutin (I-1).

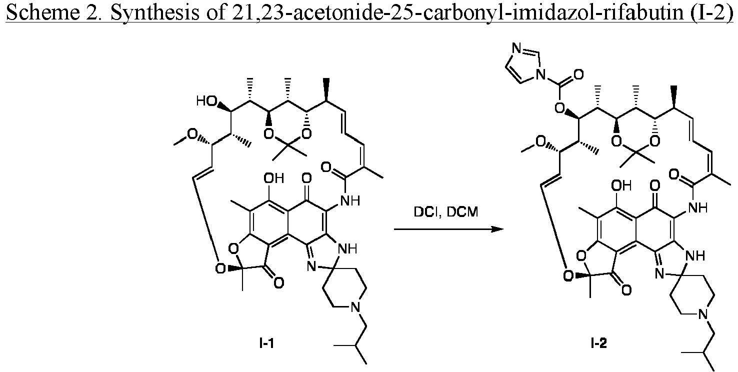

The free C25-hydroxy group of Intermediate (I-1) can be converted to a C25 imidazole carbamate (i.e., Intermediate I-2) using a carbamoylation reagent such as 1,1'-carbonyldiimidazole in a suitable solvent such as dichloromethane (DCM) as shown above in Scheme 2.

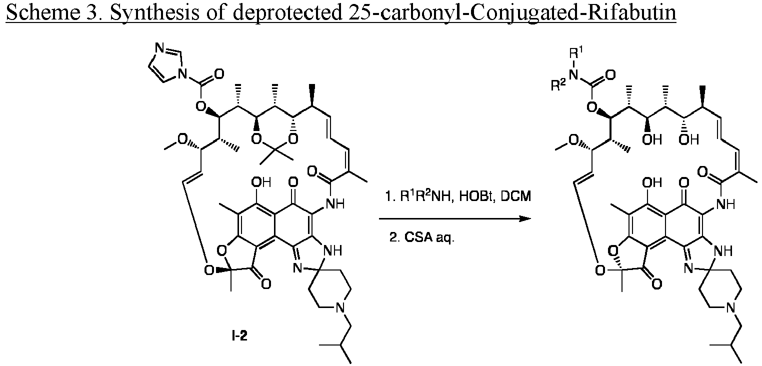

Intermediate (I-2) can be substituted by an appropriate amine in the presence of a suitable coupling reagent such as 1-hydroxy-1H-benzotriazole (HOBt). Subsequent deprotection of the C21-C23 acetal in the conjugated rifabutin can be carried out by treating with an acid such as camphorsulfonic acid in a suitable solvent such as a mixture of THF/water.

### Antibacterial Efficacy of the Disclosed Compounds

The inventive compounds are C25-modified analogs of rifabutin that exhibit good antibacterial activity against both smooth (S) and rough (R) *Mycobacterium abscessus* (*M. abscessus,* also known as "Mabs") variants. Moreover, as shown in the Examples, the inventive compounds were found to be less cytotoxic to healthy cells (e.g., HepG2 cells) compared to other known C25-analogs of rifabutin. The combination of lower cytotoxicity against healthy cells and similar or better antibacterial activity against a broad range of *M. abscessus* suggests that the inventive compounds can have a broader therapeutic window in patients than other C25-modified analogs of rifabutin.

Accordingly, in some embodiments, the inventive compounds can be used to inhibit bacterial infection, preferably infection caused by a non-tuberculous *Mycobacteria,* more preferably *M. abscessus.*

### Methods of Using the Disclosed Compounds

An aspect of the present invention relates to a compound of Formula I or a pharmaceutically acceptable salt thereof for use as a medicament.

In one aspect, the present invention provides a compound according to Formula I of the present invention or a pharmaceutically acceptable salt, tautomer, solvate or hydrate thereof, or a pharmaceutical composition comprising a compound of Formula I of the present invention, for use in a method of preventing or treating a disease in a subject, preferably an infection, further preferably a bacterial infection.

In one aspect, the present invention provides a method of treating a disease, preferably an infection, more preferably a bacterial infection in a subject in need thereof, the method comprising administering to the subject an effective amount of a compound of Formula I of the present invention or a pharmaceutically acceptable salt, tautomer, solvate, or hydrate thereof. In one aspect, the present invention provides a method of treating a disease, preferably an infection, more preferably a bacterial infection in a subject in need thereof, the method comprising administering to the subject an effective amount of a pharmaceutical composition comprising a compound of Formula I of the present invention or a pharmaceutically acceptable salt, tautomer, solvate, or hydrate thereof.

In one aspect, the present invention provides the use of a compound of Formula I of the present invention or a pharmaceutically acceptable salt, tautomer, solvate, or hydrate thereof in the manufacture of a medicament for treating a disease, preferably an infection, more preferably a bacterial infection in a subject in need thereof. In one aspect, the present invention provides the use of a pharmaceutical composition comprising a compound of Formula I of the present invention or a pharmaceutically acceptable salt, tautomer, solvate, or hydrate thereof in the manufacture of a medicament for treating a disease, preferably an infection, more preferably a bacterial infection in a subject in need thereof.

In some preferred embodiments, the bacterial infection is caused by one or more bacterium belonging a genus of non-tuberculous *Mycobacteria,* preferably *M. abscessus.*

In some preferred embodiments, the infection is caused by one or more bacterium belonging to a genus of non-tuberculosis *Mycobacterium*, preferably *M. abscessus*, *M. avium*, *M. kansasii*, *M. smegmatis*, *M. xenopi* and/or *M. malmoense*, more preferably *M. abscessus*, *M. avium*, *M. kansasii*, and/or *M. xenopi,* yet more preferably *M. abscessus.*

In some preferred embodiments, the inventive compounds are used for the treatment of a non-tuberculous *Mycobacteria* that causes a non-tuberculous *Mycobacteria* pulmonary infection. In some embodiments the non-tuberculosis *Mycobacterium* is *M. abscessus, M. avium, M. kansasii, M. smegmatis, M. xenopi* and/or *M. malmoense,* preferably *M. abscessus, M. avium, M. kansasii*, *and*/*orM. xenopi*, more preferably *M*. *abscessus.* Accordingly, in some preferred embodiments, the inventive compounds are for use in the treatment of a non-tuberculous *Mycobacteria* pulmonary infection. In some preferred embodiments, the inventive compounds are for use in the manufacture of a medicament for the treatment of a non-tuberculous *Mycobacteria* pulmonary infection. In some preferred embodiments, the present invention provides a method of treating a non-tuberculous *Mycobacteria* pulmonary infection in a subject in need thereof, comprising administering to the subject an effective amount of an inventive compound of the invention.

Illustrative pharmaceutical compositions are tablets and gelatin capsules comprising a compound of the invention and a pharmaceutically acceptable carrier, such as a) a diluent, e.g., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, algiic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

The inventive compounds and pharmaceutical compositions may be administered by any suitable route, e.g. orally, for example as a syrup, tablet, capsule, controlled-release preparation, fast-dissolving preparation, or lozenge.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

The disclosed compounds can be also formulated as a suppository that can be prepared from fatty emulsions or suspensions, using polyalkylene glycols such as propylene glycol, as the carrier.

The disclosed compounds can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

Another aspect of the invention relates to a pharmaceutical composition comprising a compound of the present invention and a pharmaceutically acceptable carrier. The pharmaceutically acceptable carrier can further include an excipient, diluent, or surfactant. Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

The dosage regimen utilizing the disclosed compound is selected in accordance with a variety of factors including type, species, age, weight, sex and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter, or arrest the progress of the condition.

Effective dosage amounts of the disclosed compounds, when used for the indicated effects, range from about 0.5 mg to about 5000 mg of the disclosed compound as needed to treat the condition. Compositions for *in vivo* or *in vitro* use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

### Equivalents

While the present technology has been described in conjunction with the specific embodiments set forth above, many alternatives, modifications and other variations thereof will be apparent to those of ordinary skill in the art. All such alternatives, modifications and variations are intended to fall within the spirit and scope of the present invention.

### EXAMPLES

The invention will now be illustrated by way of the following non-limiting examples. While particular embodiments of the invention are described below, a skilled person will appreciate that various changes and modifications can be made. References to preparations carried out in a similar manner to, or by the general method of, other preparations, may encompass variations in routine parameters such as time, temperature, workup conditions, minor changes in reagents amounts, and the like.

### Abbreviations

The following list provides definitions of certain abbreviations and symbols as used herein. It will be appreciated that the list is not exhaustive, but the meaning of those abbreviations and symbols not herein below defined will be readily apparent to those skilled in the art. In describing the invention, chemical elements are identified in accordance with the Periodic Table of the Elements.
- ACN: acetonitrile
- AcOH: acetic acid
- aq.: aqueous
- Ar: argon
- CSA: camphorsulphonic acid
- DCM: dichloromethane
- DMF: dimethylformamide
- eq.: equivalent
- EtOAc: ethyl acetate
- EtOH: ethanol
- h: hour(s)
- HOBt: 1-hydroxy-1H-benzotriazole
- HPLC: high performance liquid chromatography
- LC: liquid chromatography
- M: molar
- MeOH: methanol
- MS: mass spectroscopy
- min: minutes
- NMR: nuclear magnetic resonance spectroscopy
- rt: room temperature
- Rt: retention time
- sat.: saturated

Unless specified otherwise, the purity and identity of Intermediate or example compounds were assessed by state-of-the-art HLPC-MS. Methods are described below.

### Characterization of the Compounds

Method A or B was used for the intermediates. Method A or B was used to measure final purity of the compounds as noted herein. Purity (%) was determined by reversed phase HPLC or UPLC, using UV detection (254 or 214 nm). Structure was confirmed by MS, using electro spray ionization positive (ESI+) method and reported as [M+H]⁺, referring to the protonated molecular ion.

### Method A:

UPLC system: UPLC I BIN SOL MGR with ACQUITY UPLC I-Class eK PDA Detector; Column: Acquity BEH C18 column (1.7µm particle size, dimensions 50mm × 2.1mm); Mobile phases: phase A (H₂O/ammonium formate, pH 3.75 (A) or 9.2 (B)) and phase B (CH₃CN + 5% H₂O/ammonium formate, pH 3.75 (A) or 9.2 (B)) were used according to the following methods:

| Method A | | | |
|---|---|---|---|
| Time (min) | Phase A | Phase B | Flow (mL/min) |
| 0.00 | 98.0 | 2.0 | 0.6 |
| 0.20 | 98.0 | 2.0 | 0.6 |
| 2.50 | 0.0 | 100.0 | 0.6 |
| 3.50 | 0.0 | 100.0 | 0.6 |
| 3.60 | 98.0 | 2.0 | 0.6 |
| 5.00 | 98.0 | 2.0 | 0.6 |

Mass spectrometer: ACQUITY QDa (Performance) Xevo TQD. Ionization: electrospray (polarity: negative and positive). Unless otherwise stated, analyses lasted five minutes.

### Method B:

Waters LC-H class plus, QDa; Column Acquity HSS-T3 (2.1 × 100mm, 1.8 µm);
Mobile phases: Phase A: 0.1% Trifluoroacetic Acid in Water, Phase B: Acetonitrile were used according to the following gradient:

| Method B - 10 min | | | |
|---|---|---|---|
| Time | Phase A | Phase B | Flow (ml/min) |
| 0.0 | 90.0 | 10.0 | 0.3 |
| 1.0 | 90.0 | 10.0 | 0.3 |
| 2.0 | 85.0 | 15.0 | 0.3 |
| 4.5 | 45.0 | 55.0 | 0.3 |
| 6.0 | 10.0 | 90.0 | 0.3 |
| 8.0 | 10.0 | 90.0 | 0.3 |
| 10.0 | 10.0 | 90.0 | 0.3 |

| Method B - 15 min | | | |
|---|---|---|---|
| Time | Phase A | Phase B | Flow (ml/min) |
| 0.0 | 90.0 | 10.0 | 0.3 |
| 1.0 | 90.0 | 10.0 | 0.3 |
| 2.0 | 85.0 | 15.0 | 0.3 |
| 4.5 | 70.0 | 30.0 | 0.3 |
| 6.0 | 55.0 | 45.0 | 0.3 |
| 8.0 | 20.0 | 80.0 | 0.3 |
| 10.0 | 10.0 | 90.0 | 0.3 |
| 12.0 | 90.0 | 10.0 | 0.3 |
| 15.0 | 90.0 | 10.0 | 0.3 |

### NMR Analysis

NMR spectra were recorded on a Bruker DRX-300 spectrometer or Bruker 500 MHz spectrometer with a TXI probe or Bruker 400 MHz. Chemical shifts are in parts per million (ppm). The assignments were made using one-dimensional (1D) ¹H and ¹³C spectra and two-dimensional (2D) HSQC, HMBC spectra.

The compounds of the current invention were obtained from the free 25-OH rifabutin (Intermediate **I-1**) prepared from commercially available rifabutin as shown below.

### EXAMPLE 1: Synthesis of 21,23-acetonide-25-hydroxy-rifabutin (I-1)

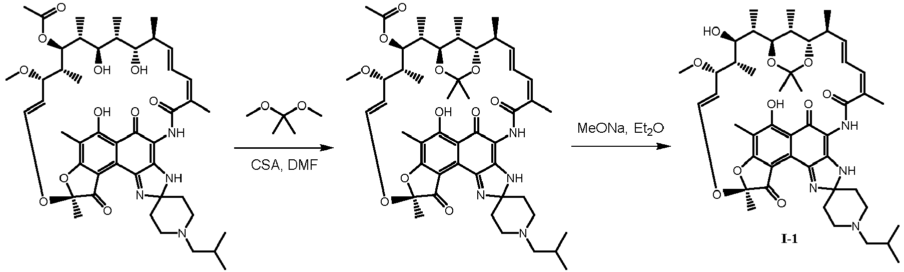

### Synthesis of Intermediate I-1:

### Step 1:

Commercially available Rifabutin (40.0 g, 47.2 mmol) was dissolved in dry DMF (80 mL) at RT under nitrogen atmosphere. 2,2-Dimethoxy-propane (58.1 mL, 472 mmol) and (1R) (-) 10 camphorsulfonic acid (12.6 g, 54.3 mmol) were sequentially added to the solution. The reaction mixture was stirred at RT for 26 h under inert atmosphere. The mixture was then cooled to 0°C and poured into a mixture of a solution of NaHCO₃ (sat. aq., 700 mL) and water (500 mL). The reaction flask was washed with acetone (100 mL). The resulting suspension was stirred in an ice bath for 10 min and filtered. The cake was rinsed with a solution of NaHCO₃ (sat. aq., 100 mL) and water (50 mL) and dried under vacuum at 40 °C for 24 h. The crude product was purified by flash chromatography (DCM to 50% of the mixture DCM/MeOH/NH₄OH 90/9/1.5 in DCM) to afford the 21,23-acetonide-rifabutin as a purple solid (37.68 g, 90 % yield).

LC/MS A (ESI+): tr = 3.20 min, m/z [M+H]⁺ = 887.48, UV purity: 97 % (254 nm). ¹H NMR (300 MHz, CDCl₃): *δ* 14.82 (s, 1H), 8.76 (brs, 1H), 7.75 (s, 1H), 6.28 (dd, *J* = 15.5, 10.7 Hz, 1H), 6.16 (dd, *J* = 10.7, 1.4 Hz, 1H), 6.08 (dd, *J* = 15.5, 6.8 Hz, 1H), 5.87 (d, J = 12.2 Hz, 1H), 5.08 (dd, *J* = 12.1, 6.7 Hz, 1H), 4.91 (d, *J* = 7.5 Hz, 1H), 3.58 (dd, J = 3.1 Hz, 1H), 3.34 (d, *J* = 6.7 Hz, 1H), 3.09-2.91 (m overlapped, 2H), 3.06 (dd overlapped, *J* = 10.3, 5.4 Hz, 1H), 2.79 (s, 3H), 2.72-2.52 (m, 2H), 2.35-2.22 (m overlapped, 3H), 2.31 (s overlapped, 3H), 2.20-1.73 (m overlapped, 6H), 2.04 (s overlapped, 3H), 1.96 (s overlapped, 3H), 1.77 (s overlapped, 3H), 1.55-1.36 (m, 2H), 1.23 (s, 3H), 0.93 (d, *J* = 6.6 Hz, 6H), 0.88-0.80 (m, 9H), 0.69 (d, *J* = 7.1 Hz, 3H), 0.40 (d, *J* = 7.1 Hz, 3H).

¹³C NMR (75 MHz, CDCl₃): *δ* 192.8, 181.3, 172.2, 170.6, 169.0, 168.3, 155.2, 142.3, 140.9, 140.8, 132.7, 131.3, 125.5, 123.8, 115.4, 113.7, 111.7, 108.8, 106.2, 104.6, 100.2, 95.0, 79.0, 76.9, 74.7, 71.2, 66.4, 56.2, 51.6 (2C), 41.4, 40.8, 36.9, 36.1, 35.5, 34.2, 25.9 (2C), 23.6, 21.0 (3C), 20.4, 20.3, 18.0, 12.9, 10.0, 9.1, 7.9.

### Step 2:

21,23-acetonide-rifabutin (10.6 g, 11.9 mmol) was dissolved in dry diethyl ether (500 mL). The solution was cooled to -10°C and degassed with Ar bubbling. After 15 min, a solution of NaOMe (25wt% in MeOH, 30 mL) was slowly added. Precipitation occurred and another portion of diethyl ether (100 mL) was added to homogenize the mixture. Subsequently, another portion of NaOMe solution was added (25wt% in MeOH, 27.4 mL). The solution was stirred at -5°C for 10 min before the ice bath was removed then the reaction mixture was stirred at RT for 6 h. A solution of NaHCO₃ (sat. aq., 400 mL) was added and the layers were separated. The aqueous layer was extracted with diethyl ether (400 mL) and the combined organic layers were washed with NaCl brine (300 mL) and evaporated to afford the desired crude product as a black-purple powder. The product was purified by flash chromatography (DCM to 50% of the mixture DCM/MeOH/NH₄OH 90/9/1.5 in DCM). After evaporation, the product was redissolved in acetone and slowly added into water under vigorous stirring. The solid was collected by filtration and dried at 40°C to yield 8.54 g of the Intermediate **I-1**.

LC/MS A (ESI+): tr = 2.98 min, m/z [M+H]⁺ = 845.59, UV purity: 97 % (254 nm). ¹H NMR (300 MHz, CDCl₃): *δ* 14.81 (s, 1H), 8.64 (brs, 1H), 7.72 (s, 1H), 6.25 (dd, *J* = 15.6 Hz, 10.3 Hz, 1H), 6.12 (dd overlapped, *J* = 10.2 Hz, 1.2 Hz, 1H), 6.11 (d overlapped, *J* = 10.3 Hz, 1H), 5.94 (dd, J = 15.6 Hz, 5.6 Hzn 1H), 4.93 (dd, *J* = 12.2 Hz, 9.2 Hz, 1H), 3.55 (dd, *J* = 9.2 Hz, 3.3 Hz, 1H), 3.48 (dd, *J* = 10.4 Hz, 3.0 Hz, 1H), 3.42 (d, *J* = 8.7 Hz, 1H), 3.31 (brs, 1H), 3.14-3.06 (m overlapped, 1H), 3.10 (s overlapped, 3H), 3.00-2.86 (m, 2H), 2.71-2.47 (m, 2H), 2.31-2.17 (m overlapped, 3H), 2.2 (s overlapped, 3H), 2.11-1.77 (m overlapped, 5H), 2.01 (s overlapped, 3H), 1.73 (s, 3H), 1.67-1.57 (m, 1H), 1.57-1.44 (m, 1H), 1.33-1.25 (m, 1H), 1.04 (s, 3H), 0.89 (d, *J* = 6.5 Hz, 6H), 0.84-0.76 (m, 9H), 0.71 (d, *J* = 6.8 Hz, 3H), 0.48 (d, *J* = 7.0 Hz, 3H).

¹³C NMR (75 MHz, CDCl₃): *δ* 191.3, 181.6, 171.4, 169.2, 168.2, 155.2, 142.7, 142.6, 140.3, 132.4, 132.0, 125.5, 124.2, 114.3, 111.9, 111.8, 108.6, 105.7, 104.9, 99.6, 95.0, 83.0, 75.5, 71.5, 71.0, 66.4, 56.2, 51.8, 51.4, 41.1, 39.7, 36.4, 35.3 (2C), 34.6, 25.9, 25.6, 24.3, 21.01, 20.97, 20.0, 19.9, 17.8, 12.9, 8.4, 7.8.

### EXAMPLE 2: Synthesis of 25-carbonyl-imidazol-21.23-acetonide-Rifabutin (I-2)

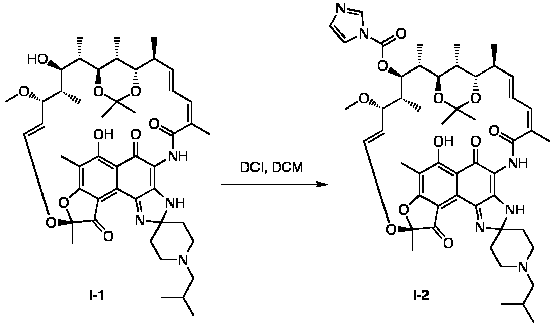

Intermediate I-1 (1.00 g, 1.18 mmol, 1 eq) was dissolved in anhydrous DCM (40ml) under inert atmosphere. To this solution was added carbonyldiimidazole (1.92 g, 11.80 mmol, 10 eq) and the reaction was stirred at RT. After 16 h, the mixture was washed with an aqueous saturated solution of NaHCO₃ (40ml) and NaCl brine (40mL). The organic layer was dried over MgSO₄, filtered, and DCM was evaporated under vacuum. The obtained product was purified by flash chromatography with cyclohexane/Acetone (from 100/0 to 60/40 over 20 CV) to yield 25-carbonyl-imidazol-21,23-acetonide-Rifabutin as a red solid (m= 660mg, yield=59%).

LC/MS A (ESI+): tr = 2.29 min, m/z [M+H]⁺ = 939.52, UV purity: 99% (254 nm).

¹H NMR (300 MHz, CDCl₃): δ (ppm) 14.78 (s, 1H), 8.92-8.46 (brs, 1H), 8.03-7.92 (m, 2H), 7.30-7.23 (m, 1H), 6.97-6.91 (m, 1H), 6.21 (dd, *J* = 15.6, 10.6 Hz, 1H), 6.07 (dd, *J* = 10.5, 1.2 Hz, 1H), 5.97 (dd, *J* = 15.6, 6.9 Hz, 1H), 5.89 (d, *J* = 12.1 Hz, 1H), 5.10-4.95 (m, 2H), 3.51 (dd, *J* = 10.5, 3.0 Hz, 1H), 3.30 (dd, *J* = 7.4, 1.7 Hz, 1H), 3.02-2.82 (m, 3H), 2.67 (s, 3H), 2.62-2.44 (m, 2H), 2.20 (s, 3H), 2.27-2.15 (m, 3H), 1.97 (s, 3H), 1.69 (s, 3H), 2.16-1.58 (m overlapped, 8H), 1.54-1.38 (m, 1H), 1.08 (s, 3H), 0.90-0.82 (m, 6H), 0.82-0.70 (m, 12H), 0.45 (d, *J* = 7.0 Hz, 1H).

¹³C NMR (75 MHz, CDCl₃): δ (ppm) 191.9, 181.2, 171.6, 168.7, 168.0, 154.9, 148.1, 142.3, 141.7, 140.0, 136.8, 132.0, 131.5, 130.3, 125.2, 123.8, 116.8, 113.7, 113.0, 111.5, 108.4, 105.7, 104.5, 99.9, 94.9, 79.5, 78.7, 76.0, 70.9, 66.2, 55.7, 51.4, 51.2, 40.6, 40.3, 36.6, 36.1, 35.3, 34.0, 26.7, 25.7, 25.4, 23.16, 20.76, 20.74, 19.9, 17.7, 12.7, 10.2, 9.4, 7.6.

### EXAMPLE 3: Synthesis of Compound 2

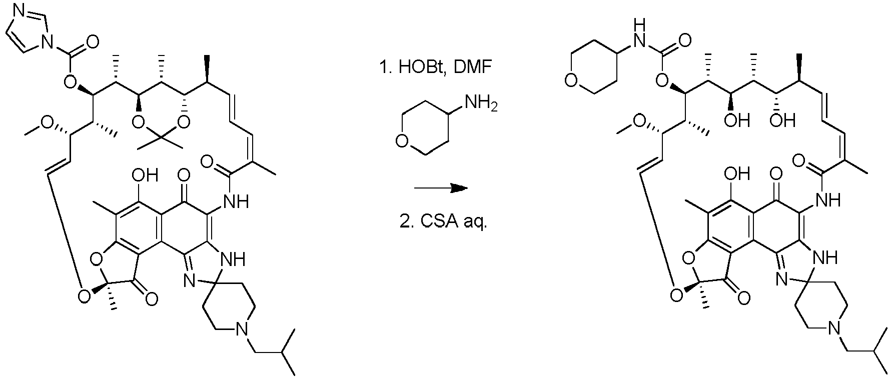

Intermediate I-2 (25-imidazo-carbonyl-21,23-acetonide-rifabutin, 1g, 1 eq.) was dissolved in DMF (0.025 mol/L). To this solution was added tetrahydro-2H-pyran-4-amine (5 eq.) and HOBt (20 eq.) and the mixture was stirred for 16h at 50°C. Then, the mixture was diluted with AcOEt, washed with sat. solution of NaHCO₃ and then brine. The combined organic phase were dried, filtered and concentrated under reduce pressure. The crude was purified by column chromatography (from 100% DCM to 90/10 DCM/MeOH). The recovered fraction was dissolved in a mixture THF/0.5 N aqueous solution of CSA and stirred overnight. Then, AcOEt was added and the organic phase was washed with sat. solution of NaHCO₃ and brine. The organic phase was dried, filtered and concentrated under reduce pressure. The crude was purified by column chromatography (from 100% DCM to 90/10 DCM/MeOH) to afford the desired product [(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-tetrahydropyran-4-ylcarbamate (Compound 2) as purple solid (228 mg).

LC/MS B (ESI+): tr = 7.83 min, m/z [M+H]⁺ = 932.71, UV purity: >95% (254 nm).

¹H NMR (300 MHz, C6D6): δ(ppm) 15.49 (s, 1H), 9.21 (s, 1H), 8.61 (s, 1H), 6.55 (dd, J = 15.7, 9.6 Hz, 1H), 6.40 (d, J = 12.5 Hz, 1H), 5.98 (d, J = 9.5 Hz, 1H), 5.91 (dd, J = 15.7, 7.2 Hz, 1H), 5.27 (dd, J = 12.6, 7.4 Hz, 1H), 5.00 (d, J = 3.7 Hz, 1H), 4.75 (d, J = 10.5 Hz, 1H), 4.14 (d, J = 8.2 Hz, 1H), 3.86 (s, 1H), 3.80 (d, J = 9.5 Hz, 1H), 3.60 (t, J = 12.2 Hz, 2H), 3.35 (m, 1H), 3.30 (m, 1H), 3.25 (m, 1H), 3.07-2.92 (m, 3H), 2.91 (s, 3H), 2.86 (m, 1H), 2.56-2.42 (m, 4H), 2.37 (m, 1H), 2.09-1.94 (m, 3H), 1.92 (s, 3H), 1.87-1.70 (m, 5H), 1.68 (s, 3H), 1.62 (m, 1H), 1.48 (m, 1H), 1.25 (m, 1H), 1.12 (d, J = 6.9 Hz, 3H), 1.01-0.86 (m, 8H), 0.76 (d, J = 6.9 Hz, 4H), 0.71 (d, J = 6.8 Hz, 4H), 0.30 (d, J = 7.1 Hz, 3H).

¹³C NMR (75 MHz, C6D6): δ(ppm) 191.77, 182.35, 171.60, 168.93, 168.35, 157.44, 155.81, 145.34, 142.74, 141.33, 132.73, 131.65, 128.35, 125.87, 124.97, 114.78, 114.39, 112.62, 109.45, 107.96, 105.91, 94.87, 81.32, 77.59, 74.70, 73.33, 66.49, 66.41, 56.60, 51.77, 51.58, 47.55, 39.10, 39.01, 38.17, 36.64, 36.11, 34.06, 33.63, 33.00, 26.05, 22.05, 20.93, 20.66, 17.38, 12.37, 11.57, 9.15, 7.88.

Compounds 1 and 3-13 were synthesized according to the same procedure used for the synthesis of Compound 2 as described below:
Intermediate I-2 (25-imidazo-carbonyl-21,23-acetonide-rifabutin, 1 eq.) was dissolved in DMF (0.025 mol/L). To this solution was added corresponding amine (5 eq.) and HOBt (20 eq.) and the mixture was stirred for 16h at 50°C. Then, the mixture was diluted with AcOEt, washed with sat. solution of NaHCO₃ and then brine. The combined organic phase were dried, filtered and concentrated under reduce pressure. The crude was purified by column chromatography (from 100% DCM to 90/10 DCM/MeOH) when needed. The recovered fraction was dissolved in a mixture THF/0.5 N aqueous solution of CSA and stirred overnight. Then, AcOEt was added and the organic phase was washed with sat. solution of NaHCO₃ and brine. The organic phase was dried, filtered and concentrated under reduce pressure. The crude was purified by column chromatography (from 100% DCM to 90/10 DCM/MeOH) or HPLC to afford the desired product as purple solid.

Table 3 below summarizes exemplary compounds of the invention (R¹ and R² groups shown) that were prepared according to the protocol for Compound 2 above. Data for compounds 1-13 given in Table 3 were obtained following the analytic method B 10 minutes (*) and 15 minutes (**) gradient. Where the starting amine was originally prepared as a hydrochloride, an excess of TEA was added prior to the addition of I-2 to neutralize the hydrochloride salt. When starting amine where not commercially available, preparation was made according to general literature protocols.

In table 3, molecular weight (MW) is defined as the sum of atomic masses of the atoms constituting the molecule as an average of the different isotopes in their natural abundance. The molecular exact mass (M) on the contrary, takes in account only specific set of isotopes, the most abundant ones (¹H for hydrogen, ¹²C for carbon, ¹⁴N for nitrogen, ¹⁶O for oxygen, ¹⁹F for fluorine, etc.). For a molecule of molecular mass > 1000 Da, as ¹³C has a natural abundance of 1,08%, the molecular weight presents in average M+1 Da resulting equal to the m/z ratio of the most abundant isotope revealed by the mass spectrum [M+H]⁺.

**Table 3: Molecular Weight (Calculated), Retention Time (Method B) and Measured Mass of Exemplary Compounds**

| Cpd | Structure (R¹ and R² groups shown) | MW | Rt | [M+H]⁺ |
|---|---|---|---|---|
| 1 | 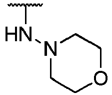 | 933.113 | 7.38** | 933.80 |
| 2 | 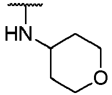 | 932.125 | 7.83** | 932.71 |
| 3 | 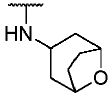 | 958.163 | 6.17* | 958.30 |
| 4 | 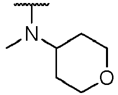 | 946.152 | 5.99* | 946.77 |
| 5 | 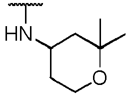 | 960.179 | 5.99* | 960.75 |
| 6 | 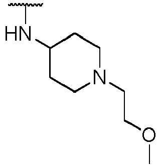 | 989.221 | 6.35* | 989.47 |
| 7 | 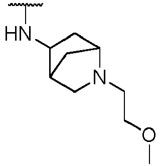 | 1001.232 | 6.92** | 1001.82 |
| 8 | 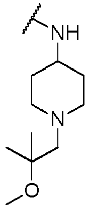 | 1,017.275 | 5.19* | 1017.79 |
| 9 | 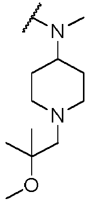 | 1,031.302 | 5.28* | 1031.86 |
| 10 | 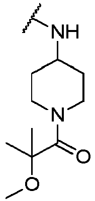 | 1,031.258 | 5.91* | 1031.86 |
| 11 | 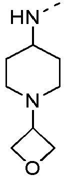 | 987.205 | 4.88* | 987.77 |
| 12 | 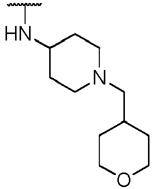 | 1029.286 | 5.00* | 1029.87 |
| 13 | 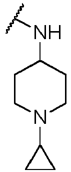 | 971.206 | 5.02* | 971.75 |

**Table 4: ¹HNMR Values of Exemplary Compounds**

| Cpd | ¹HNMR |
|---|---|
| 1 | ¹H NMR (300 MHz, C6D6): δ(ppm) 15.40 (s, 1H), 9.28 (s, 1H), 8.46 (s, 1H), 6.51 (m, 1H), 6.38 (d, *J* = 12.5 Hz, 1H), 5.94 (d, *J* = 9.1 Hz, 1H), 5.86 (dd, *J* = 15.8, 6.4 Hz, 1H), 5.29 (dd, *J* = 12.5, 8.1 Hz, 1H), 5.22 (brs, 1H), 4.67 (m, 2H), 3.71 (m, 2H), 3.40 (t, *J* = 4.2 Hz, 4H), 3.25 (m, 1H), 3.17 (s, 1H), 3.01 (m, 1H), 2.88 (s, 4H), 2.49 (s, 3H), 2.42 (m, 4H), 2.34 (m, 3H), 2.05 (d, *J* = 7.0 Hz, 2H), 1.90 (s, 3H), 1.82-1.72 (m, 5H), 1.66 (s, 3H), 1.65-1.61 (m, 2H), 1.31 (brs, 1H), 1.08 (d, *J* = 6.7 Hz, 3H), 0.92 (d, *J* = 6.2 Hz, 6H), 0.75 (d, *J* = 6.8 Hz, 3H), 0.67 (d, *J* = 6.8 Hz, 3H), 0.28 (d, *J* = 7.0 Hz, 3H). |
| 3 | ¹H NMR (300 MHz, C6D6): δ(ppm) 15.52 (s, 1H), 9.34 (s, 1H), 8.69 (s, 1H), 6.55 (dd, *J* = 15.6, 9.10 Hz, 1H), 6.41 (d, *J* = 12.5 Hz, 1H), 6.00 (d, *J* = 9.3 Hz, 1H), 5.84 (dd, *J* = 15.7, 7.0 Hz, 1H), 5.25 (dd, *J* = 12.3, 8.5 Hz, 1H), 4.99 (s, 1H), 4.85 (d, *J* = 6.4 Hz, 1H), 4.59 (d, *J* = 10.0 Hz, 1H), 4.0 (m, 1H), 3.98 (m, 1H), 3.89 (s, 1H), 3.73 (d, *J* = 9.6 Hz, 1H), 3.65 (m, 1H), 3.28 (m, 1H), 3.18 (d, *J* = 6.8 Hz, 1H), 3.00 (m, 1H), 2.90 (s, 3H), 2.86 (m, 1H), 2.51 (s, 3H), 2.47 (m, 2H), 2.35 (m, 1H), 2.05 (d, *J* = 7.1 Hz, 2H), 1.93 (s, 5H), 1.91-1.71 (m, 5H), 1.65 (s, 4H), 1.63-1.47 (m, 4H), 1.34 (m, 2H), 122 (m, 1H), 1.13 (d, *J* = 6.9 Hz, 3H), 1.06 (d, *J* = 15.4 Hz, 1H), 0.91 (d, *J* = 5.6 Hz, 6H), 0.72 (m, 6H), 0.28 (d, *J* = 6.8 Hz, 3H). |
| 4 | ¹H NMR (300 MHz, C6D6): δ(ppm) 15.49 (s, 1H), 9.22 (s, 1H), 8.63 (m, 1H), 6.53 (m, 1H), 6.37 (m, 1H), 5.96 (m, 1H), 5.87 (m, 1H), 5.20 (m, 1H), 5.09 (s, 1H), 4.77-4.64 (m, 1H), 4.07 (m, 1H), 3.93-3.70 (m, 3H), 3.20-2.97 (m, 4H), 2.89-2.80 (m, 4H), 2.58-2.45 (m, 4H), 2.41-2.32 (m, 5H), 2.07 (m, 3H), 1.91-1.70 (m, 8H), 1.70-1.62 (m, 5H), 1.40-1.28 (m, 3H), 1.20-1.09 (m, 4H), 1.05-0.83 (m, 8H), 0.77 (m, 3H), 0.66 (m, 3H), 0.26 (m, 3H). |
| 5 | ¹H NMR (300 MHz, C6D6): δ(ppm) 15.48 (s, 1H), 9.19 (s, 1H), 8.63 (s, 1H), 6.57 (m, 1H), 6.40 (dd, *J* = 12.4, 9.8 Hz, 1H), 5.99 (m, 1H), 5.91 (m, 1H), 5.28 (ddd, *J* = 13.5, 7.2 Hz, 1H), 5.03 (m, 1H), 4.76 (m, 1H), 4.10 (d, *J* = 8.1 Hz, 1H), 3.86-3.78 (m, 2H), 3.62 (m, 1H), 3.47 (m, 1H), 3.38-3.18 (m, 3H), 3.04 (m, 1H), 2.93 (s, 3H), 2.87 (m, 1H), 2.54 (m, 1H), 2.47 (d, *J* = 6.7 Hz, 3H), 2.36 (m, 1H), 2.11-2.03 (m, 2H), 1.91 (s, 3H), 1.90-1.71 (m, 5H), 1.67 (d, *J* = 4.5 Hz, 3H), 1.65-1.52 (m, 2H), 1.38-1.20 (m, 3H), 1.13 (m, 3H), 1.10 (m, 3H), 0.97 (s, 2H), 0.94-0.86 (m, 8H), 0.87-0.65 (m, 8H), 0.30 (d, *J* = 7.0 Hz, 3H). |
| 6 | ¹H NMR (300 MHz, C6D6): δ(ppm) 15.49 (s, 1H), 9.23 (s, 1H), 8.50 (s, 1H), 6.55 (dd, *J* = 15.7, 9.4 Hz, 1H), 6.40 (d, *J* = 12.4 Hz, 1H), 5.96 (d, *J* = 9.4 Hz, 1H), 5.90 (m, 1H), 5.26 (dd, *J* = 12.5, 7.6 Hz, 1H), 5.07 (d, *J* = 3.4 Hz, 1H), 7.71 (d, *J* = 10.6 Hz, 1H), 4.20 (d, *J* = 8.1 Hz, 1H), 3.85 (s, 1H), 3.77 (d, *J* = 9.4, 1H), 3.34-3.22 (m, 5H), 3.07 (s, 3H), 3.01 (m, 1H), 2.90 (s, 3H), 2.85 (m, 1H), 2.60-2.52 (m, 2H), 2.49 (s, 3H), 2.45 (m, 1H), 2.41-2.32 (m, 4H), 2.08-1.99 (m, 3H), 1.90 (s, 3H), 1.87-1.71 (m, 8H), 1.67 (s, 3H), 1.62 (m, 1H), 1.46 (m, 1H), 1.20-1.08 (m, 6H), 0.91 (d, *J* = 6.2 Hz, 6H), 0.76 (d, *J* = 6.8 Hz, 3H), 0.71 (d, *J* = 6.8 Hz, 3H), 0.31 (d, *J* = 7.0 Hz, 3H). |
| 8 | ¹H NMR (300 MHz, C6D6): δ(ppm) 15.51 (s 1H), 9.15 (s, 1H), 8.66 (s, 1H), 6.58 (dd, *J* = 15.6, 9.5 Hz, 1H), 6.40 (d, *J* = 12.4 Hz, 1H), 5.98 (d, *J* = 9.3 Hz, 1H), 5.92 (dd, *J* = 15.5, 7.0 Hz, 1H), 5.26 (dd, *J* = 12.4, 7.0 Hz, 1H), 5.14 (s, 1H), 4.77 (d, *J* = 10.3 Hz, 1H), 4.23 (s, 1H), 3.90 (m, 1H), 3.81 (d, *J* = 8.2 Hz, 1H), 3.38-3.28 (m, 3H), 3.03 (m, 1H), 2.99 (s, 3H), 2.92 (s, 3H), 2.85 (m, 1H), 2.68 (m, 2H), 2.4-2.41 (m, 5H), 2.38 (m, 1H), 2.11-1.94 (m, 7H), 1.85 (s, 3H), 1.84-1.71 (m, 6H), 1.68 (s, 3H), 1.62 (m, 1H), 1.48 (m, 1H), 1.39-1.22 (m, 1H), 1.24-1.15 (m, 2H), 1.12 (d, *J* = 6.9 Hz, 3H), 1.05 (s, 6H), 0.91 (d, *J* = 6.2 Hz, 6H), 0.76 (d, *J* = 6.8 Hz, 3H), 0.71 (d, *J* = 6.8 Hz, 3H), 0.30 (d, *J* = 7.0 Hz, 3H). |
| 10 | ¹H NMR (300 MHz, C6D6): δ(ppm) 15.49 (s, 1H), 9.23 (s, 1H), 8.62 (s, 1H), 6.56 (dd, *J* = 15.7, 9.8 Hz, 1H), 6.40 (d, *J* = 12.6 Hz, 1H), 5.96 (d, *J* = 9.4 Hz, 1H), 5.89 (dd, *J* = 15.6, 7.2 Hz, 1H), 5.30 (dd, *J* = 12.3, 7.4 Hz, 1H), 4.95 (s, 1H), 4.77 (d, *J* = 9.9 Hz, 1H), 4.44 (brs, 2H), 4.07 (d, *J* = 8.2 Hz, 1H), 3.80 (m, 2H), 3.28 (m, 3H), 3.01 (m, 1H), 2.94 (s, 3H), 2.89 (s, 3H), 2.85 (m, 1H), 2.56-2.41 (m, 5H), 2.35 (m, 1H), 2.07 (m, 1H), 2.03 (d, *J* = 7.0 Hz, 2H), 1.91 (s, 3H), 1.85-1.68 (m, 5H), 1.67 (s, 3H), 1.61 (m, 1H), 1.51 (m, 1H), 1.39 (s, 6H), 1.37-1.27 (m, 3H), 1.12 (d, *J* = 6.9 Hz, 3H), 0.99-0.79 (m, 9H), 0.76 (d, *J* = 6.8 Hz, 3H), 0.72 (d, *J* = 6.8 Hz, 3H), 0.30 (d, *J* = 7.9 Hz, 3H). |
| 11 | ¹H NMR (300 MHz, C6D6): δ(ppm) 15.52 (s, 1H), 9.26 (s, 1H), 8.54 (s, 1H), 6.53 (dd, *J* = 15.5, 9.2 Hz, 1H), 6.40 (d, *J* = 12.5 Hz, 1H), 5.97 (d, *J* = 9.2 Hz, 1H), 5.89 (dd, *J* = 15.6, 7.2 Hz, 1H), 5.27 (dd, *J* = 12.5, 7.9 Hz, 1H), 5.01 (d, *J* = 3.8 Hz, 1H), 4.68 (d, *J* = 10.5 Hz, 1H), 4.36 (m, 2H), 4.32 (m, 2H), 4.22 (d, *J* = 8.4 Hz, 1H), 3.83 (s, 1H), 3.75 (d, *J* = 9.4 Hz, 1H), 3.35-3.18 (m, 3H), 3.01 (m, 1H), 2.93 (s, 3H), 2.88 (m, 2H), 2.50 (s, 3H), 2.45 (m, 1H), 2.36 (m, 1H), 2.11-1.96 (m, 5H), 1.91 (s, 3H), 1.90-1.85 (m, 2H), 1.84-1.72 (m, 3H), 1.67 (s, 5H), 1.62 (m, 1H), 1.37 (m, 2H), 1.29 (m, 2H), 1.13 (d, *J* = 6.9 Hz, 3H), 1.09-0.95 (m, 2H), 0.91 (d, *J* = 6.2 Hz, 6H), 0.75 (d, *J* = 6.8 Hz, 3H), 0.72 (d, *J* = 6.8 Hz, 3H), 0.32 (d, *J* = 7.0 Hz, 3H). |

### EXAMPLE 4: Antibacterial Activity

MIC for *M. abscessus* smooth (S) or rough (R) was determined in standard cation adjusted Mueller Hinton broth (see CAMHB) and supplemented with 50% Human Serum (see HS).

From a fresh culture plate, cells were resuspended in 0.9% (w/v) saline solution and bacterial inoculum was prepared in the respective testing medium with 5×10⁵ CFU/mL. The appropriate volumes of a 10 mg/mL compound solution were directly dispensed in the 96-well assay plate using a digital dispenser to create two-fold dilution series from 16 to 0.001 µg/mL for *M. abscessus.* 100 µL of bacterial suspension was finally added to the compounds. Plates were covered and incubated without shaking at 30 °C for 4 days for *M. abscessus.* Every experiment contained an antibiotic as quality control. MIC was determined visually as the lowest concentration of a compound that prevents visible growth of the bacteria. The *in vitro* activity of compounds described herein was determined against *M. abscessus* (ATCC 19977). The MIC values, given in µg/mL, are given in Table X, below

Structures of comparison compounds C1, C2, C3 and C4 are also shown in the Table 5, below.

**Table. 5, In vitro activity of embodied compounds against AT. abscessus smooth (S) and rough (R) and with 50% Human Serum (HS)**

| | MIC against *M*. *abscessus* (Mabs) (µg/mL) | | | |
|---|---|---|---|---|
| Compound | S (CAMHB) | S (HS) | R (CAMHB) | R (HS) |
| 1 | 2 | 1 | 0.5 | 1 |
| 2 | 0.5 | 0.5 | 0.25 | 0.5 |
| 3 | 0.5 | 2 | 0.06 | 1 |
| 4 | 0.5 | 4 | 0.125 | 2 |
| 5 | 0.25 | 1 | 0.25 | 0.5 |
| 6 | 1 | 2 | 0.25 | 2 |
| 7 | 4 | 2 | 1 | 1 |
| 8 | 0.5 | 1 | 0.06 | 0.5 |
| 9 | 0.25 | 0.5 | 0.25 | 0.5 |
| 10 | 1 | 2 | 0.25 | 1 |
| 11 | 2 | 4 | 0.125 | 2 |
| 12 | 1 | 2 | 0.25 | 16 |
| 13 | 0.25 | 0.5 | 0.03 | 1 |
| 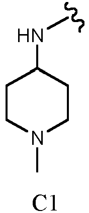 | 1 | 0.5 | 0.125 | 2 |
| 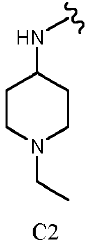 | 2 | 1 | 0.25 | 2 |
| 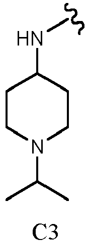 | 2 | 2 | 0.25 | 2 |
| 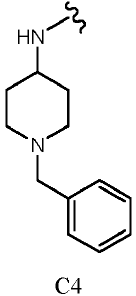 | 0.25 | 1 | 0.06 | 0.25 |
| 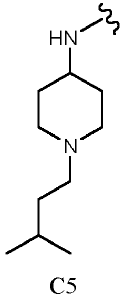 | 0.25 | 0.25 | 0.016 | 1 |

### EXAMPLE 5: Activity Against Healthy Cells

CellTiter-Blue Assay was performed to determine cytotoxicity (IC₅₀ value) of compounds against HepG2 cells (ATCC, HB-8065). IC₅₀ was determined in EMEM supplemented with 2mM Glutamine, non-essential amino acids, 10% fetal bovine serum and 1% penicillin/streptomycin. The IC₅₀ values, in µg/mL, are given in Table 6, below.

**Table 6. Activity of Exemplary Compounds against HepG2 cells**

| Compound | IC₅₀ (HepG2, µg/mL) |
|---|---|
| 1 | 99.6 |
| 2 | 70 |
| 3 | 35.49 |
| 4 | 47.2 |
| 5 | 58.4 |
| 6 | 7.5 |
| 7 | 15.7 |
| 8 | 16 |
| 9 | 10.9 |
| 10 | 46 |
| 11 | 67.1 |
| 12 | 14.7 |
| 13 | 11.4 |
| C1 | 20.29 |
| C2 | 15.1 |
| C3 | 15.76 |
| C4 | 12.1 |
| C5 | 5.7 |

## Claims

1. A compound of Formula I or a pharmaceutically acceptable salt, tautomer, solvate, hydrate or enantiomer thereof: 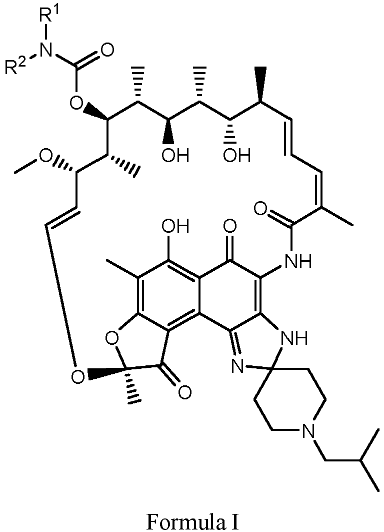 wherein:
R¹ is -H or -C₁-C₆ alkyl;
R² is a group of Formula II: 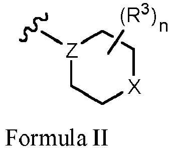 wherein
n is an integer from 0 to 8;
R³ is independently, at each occurrence, C₁-C₆ alkyl; or two R³, together with the carbon atoms to which they are attached, optionally combine to form a bridging alkylene, preferably a bridging C₁-C₂ alkylene;
X is O or NR⁴;
Z is CH or N;
R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

2. The compound of claim 1, wherein R¹ is hydrogen or methyl.

3. The compound of claim 1 or claim 2, wherein n is an integer from 0 to 4, preferably from 0 to 2.

4. The compound of any one of claims 1-3, wherein said R³ is independently, at each occurrence, C₁-C₄ alkyl.

5. The compound of any of claims 1-4, wherein X is O.

6. The compound of any of claims 1-4, wherein X is NR⁴.

7. The compound of any of claims 1-6, wherein Z is N.

8. The compound of any of claims 1-6, wherein Z is CH.

9. The compound of any of claims 1 to 4, wherein R² is selected from the group consisting of tetrahydropyran, morpholine, and 8-oxabicyclo[3.2.1]octane;
wherein said tetrahydropyran is bonded at a 4-carbon of said tetrahydropyran; said morpholine is bonded at a 4-nitrogen of said morpholine; and said 8-oxabicyclo[3.2.1]octane is bonded at a 3-carbon of said 8-oxabicyclo[3.2.1]octane;
wherein each tetrahydropyran, morpholine, and 8-oxabicyclo[3.2.1]octane is independently optionally substituted with one or more R³; and
R³ is independently, at each occurrence, C₁-C₆ alkyl.

10. The compound any of claims 1 to 4, wherein R² is piperidine or 2-azabicyclo [2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2.1]heptane is independently substituted at the 2-nitrogen with R⁴;
R⁴ is independently, at each occurrence, selected from 3- to 5-membered heterocycloalkyl; -C(O)C₁-C₆ alkyl, wherein said C₁-C₆ alkyl is optionally substituted with one or more C₁-C₆ alkoxy; -C₂-C₆ alkyl, wherein said C₂-C₆ alkyl is substituted with one or more C₁-C₆ alkoxy; -CH₂-tetrahydropyran; and cyclopropyl.

11. The compound of any of claims 9-10, wherein R² is piperidine or 2-azabicyclo[2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2.1]heptane is independently substituted at the 2-nitrogen with R⁴;
R⁴ is independently, at each occurrence, selected from 3- to 5-membered heterocycloalkyl; and -C(O)C₁-C₆ alkyl, wherein said C₁-C₆ alkyl is optionally substituted with one or more C₁-C₆ alkoxy.

12. The compound of any of claims 9-11, wherein R² is piperidine or 2-azabicyclo[2.2.1]heptane,
wherein said piperidine is bonded at a 4-carbon of said piperidine, and said 2-azabicyclo[2.2.1]heptane is bonded at a 5-carbon of said 2-azabicyclo[2.2.1]heptane;
wherein each piperidine is independently substituted at the 1-nitrogen with R⁴, and wherein each 2-azabicyclo[2.2.1]heptane is independently substituted at the 2-nitrogen with R⁴;
R⁴ is independently, at each occurrence, selected from -C₂-C₆ alkyl, wherein said C₂-C₆ alkyl is substituted with one or more C₁-C₆ alkoxy; -CH₂-tetrahydropyran; and cyclopropyl.

13. The compound of claim 1, wherein said compound is selected from:
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-morpholinocarbamate; [(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-tetrahydropyran-4-ylcarbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-(8-oxabicyclo[3.2.1]octan-3-yl)carbamate;
[(7S,9E,11S,12R,13S,14R,15R,16R,17S,18S,19E,21Z)-2,15,17-trihydroxy-1'-isobutyl-11-methoxy-3,7, 12, 14, 16, 18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-methyl-N-tetrahydropyran-4-yl-carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-(2,2-dimethyltetrahydropyran-4-yl)carbamate;
[(7S,9E,11S,12R,13S,14R,15R,16R,17S,18S,19E,21Z)-2,15,17-trihydroxy-1'-isobutyl-11-methoxy-3,7, 12, 14, 16, 18,22-heptamethyl-6,23,32-trioxo-spiro[8,33-dioxa-24,27,29-triazapentacyclo[23.6.1.14,7.05,31 026,30]tritriaconta-1(31),2,4,9,19,21,25,29-octaene-28,4'-piperidine]-13-yl] N-[1-(2-methoxyethyl)-4-piperidyl] carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-[2-(2-methoxyethyl)-2-azabicyclo [2.2.1]heptan-5-yl]carbamate;
[(7S,9E,11S,12R,13S,14R,15R,16R,17S,18S,19E,21Z)-2,15,17-trihydroxy-1'-isobutyl-11-methoxy-3,7, 12, 14, 16, 18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-[1-(2-methoxy-2-methylpropyl)-4-piperidyl]carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-[1-(2-methoxy-2-methylpropyl)-4-piperidyl]-N-methyl-carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro [8,33-dioxa-24,27,29-triazapentacyclo[23.6.1.14,7.05,31.026,30]tritriaconta-1(31),2,4,9,19,21,25,29-octaene-28,4'-piperidine]-13-yl] N-[1-(2-methoxy-2-methylpropanoyl)-4-piperidyl]carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-[1-(oxetan-3-yl)-4-piperidyl] carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-[1-(tetrahydropyran-4-ylmethyl)-4-piperidyl]carbamate;
[(7S,9E,11S,12R,13S,14R,15R,16R,17S,18S,19E,21Z)-2,15,17-trihydroxy-1'-isobutyl-11-methoxy-3,7,12,14,16,18,22-heptamethyl-6,23,32-trioxo-spiro[8,33-dioxa-24,27,29-triazapentacyclo[23.6.1.14,7.05,31.026,30]tritriaconta-1(31),2,4,9,19,21,25,29-octaene-28,4'-piperidine]-13-yl] N-(1-cyclopropyl-4-piperidyl)carbamate; or a pharmaceutically acceptable salt thereof.

14. A pharmaceutical composition comprising at least one compound according to any of the preceding claims, or a pharmaceutically acceptable salt, tautomer, solvate or hydrate thereof, and a pharmaceutically acceptable excipient.

15. A compound according to any of the claims 1 to 12 or a pharmaceutically acceptable salt, tautomer, solvate or hydrate thereof, for use as a medicament.
